# EUROPEAN PATENT APPLICATION

(11) **EP 4 249 592 A2**
(43) Date of publication of application: **27.09.2023**
(21) Application number: 23176208.9
(22) Date of filing: 15.07.2022
(51) Int. Cl.: C12N 5/10

(54) **IDENTIFICATION OF COMMON TUMOR-SPECIFIC T CELL RECEPTORS AND ANTIGENS**

(30) Priority: 15.07.2021 EP 21185876
(62) Divisional of application: 22777948.5
(71) Applicant: Boehringer Ingelheim International GmbH, 55216 Ingelheim am Rhein (DE)
(72) Inventor: Hennig, Steffen, 10961 Berlin (DE)
(74) Representative: Schulz Junghans Patentanwälte PartGmbB

(57) **Abstract**

The present invention relates to a method for identification of common patient-spanning tumor-specific T cell receptors (TCRs) and their corresponding antigens. The invention also relates to these TCR sequences, a nucleic acid encoding the TCR, and a T cell comprising the TCR and/or the encoding nucleic acid.

## Description

The present invention relates to a method for identification of common patient-spanning tumor-specific T cell receptors (TCRs) and their corresponding antigens. The invention also relates to these TCR sequences, a nucleic acid encoding the TCR, and a T cell comprising the TCR and/or the encoding nucleic acid.

### Background of the Invention

Ever since it has been shown that the immune system is capable of combatting and rejecting tumors, great efforts have been made to develop therapeutic or preventative cancer vaccines. These efforts were met with formidable challenges in antigen discovery since suitable tumor antigens for vaccination need to combine three basic requirements. They have to be immunogenic to elicit an efficacious therapeutic response. They need to be tumor-specific to allow a safe treatment, especially in the preventative setting. However, a cross-reactivity with pathogen-associated antigens is within the scope of the invention. Finally, there is the need to identify shared antigens which are expressed in tumors of many patients.

In the last decade two high throughput platforms were developed and extensively used in tumor antigen discovery. Both, however, fulfill the requirements only to a limited extent. Whole exome sequencing approaches clearly allow the identification of tumor-specific mutated antigens. But except certain recurrent driver mutations the vast majority of identified mutation reactive antigens are patient specific. On the other hand, mass spectroscopy-based approaches are able to detect shared HLA-presented peptides in tumors of different patients. But proof of immunogenicity and tumor-specificity for those peptides remain challenging tasks.

Adoptive cell therapy (ACT) with T cells genetically engineered to express tumor-reactive chimeric antigen receptors (CAR-T cells) or T-cell receptors (TCRs) is a promising treatment strategy for patients with cancer. In contrast to hematologic malignancies where CAR-T cells against certain lineage-specific cell surface antigens have been approved because of their high efficacy with manageable side effects, for solid cancers, the application of CAR-T cells is (currently) not feasible due to the lack of cell surface target antigens with tumor-restricted expression. (Patient) T cells genetically engineered to express tumor-specific transgenic TCRs (tsTCRtg-T cells) recognizing peptides from tumor-associated or tumor-specific antigens (TAA or TSA) presented by HLA-molecules (pMHC) represent attractive alternatives. While TAA (e.g. Cancer/Germline-, differentiation-, overexpressed antigens) and viral (v)TSA (in tumors with viral etiology) can be widely shared between tumors and result in the presentation of common pMHC in HLA-matched patients, the vast majority of (non-viral) TSA (neoantigens) are unique to individual cancers. The resulting vast variety of pMHC have to be regarded as private antigens of individual subjects. However, in a small number of cases, TSA resulting from point mutations or chromosomal translocations that affect common driver genes of malignancy and are shared between tumors have been shown to be immunogenic (e.g. RAS-, TP53-, BRAF-, PIK3CA-mutations and translocations involving ALK, ROS, NTRK, RET, etc.). Also, thus far less well-defined antigen categories like tumor-specific cryptic ("dark matter") or aberrantly spliced transcripts, have the potential to be shared between tumors and recognized by T cells.

Previously, the inventors have developed a method that identifies tumor-specific T-cell receptors by comparing CDR3 sequences obtained from TILs with T-cells in the adjacent tissue (WO 2017/025564 A1). Thus, it is possible to delineate tumor-specificity by the increased presence of T-cell clones in the tumor vs. non-tumor of a patient. However, most tumor-specific antigens arise through mutations that are limited to the individual patient. Whereas private neoantigens can be targeted by personalized tsTCRtg-T cell therapies, shared TAA or TSA are ideal targets for off-the-shelf tsTCRtg-T cell therapies in patients with expression of matched HLA alleles. Personalized therapy is time-consuming, costly and highly regulated by FDA and EMA (ATMP, advanced medicinal products; gene therapy medicinal products). In addition, many patients' diseases progress faster than personalized therapeutics can be produced. Therefore, it would be highly advantageous to develop a method that identifies carriers of such common tumor-specific TCRs by scanning their TIL-repertoires for identical or highly similar antigen-recognition domains (CDR3α and -β) thereby providing off-the-shelf therapeutic receptors and concomitantly opening up an opportunity to identify the shared tumor-specific antigens for additional therapeutic options.

Based on the above-mentioned state of the art, the objective of the present invention is to provide means and methods to identify common tumor-specific TCR sequences and their corresponding antigens. This objective is attained by the subject-matter of the independent claims of the present specification, with further advantageous embodiments described in the dependent claims, examples, figures and general description of this specification.

### Summary of the Invention

Rather than starting with antigen candidates that need critical validations in specificity and functional tests, an alternative way is to analyze T cell repertoires in tumors of different cancer patients searching for specific effects originating from shared tumor antigens. When a T-cell infiltrates the tumor and provokes a specific receptor mediated interaction with a tumor antigen, this encounter is followed by activation, proliferation and enrichment of the clone in the tumor. Thus, the preferred localization of this unique TCR clonotype, as determined quantitatively by the ratio of the TCR clonotype frequencies between tumor and adjacent non-tumor tissue is a predictor of tumor-specificity. This technology is described in WO 2017/025564 A1. If such a unique tumor-specific TCR clonotype or structurally closely related TCR clonotypes, referred to as a TCR cluster, are detected in the tumors of other patients, this is indicative of the existence of a shared tumor antigen in these patients. This is particularly informative when TCR clusters are detected in HLA-matched patients revealing the nature of the HLA allele presenting the shared antigenic epitope. As the last step, complete elucidation of the cluster TCRs, e.g. by single cell technologies, will yield α/β-TCRs with specificity for the shared antigen.

Such HLA restricted α/β-TCRs with specificity for shared tumor antigens are the starting point of important applications.

As unprecedented novel tools they can be used as specific probes in antigen discovery guiding the targeted search for shared tumor antigens.

As "off the shelf" TCRs in vector form they can be used for transduction into autologous T cells of cancer patients for immunotherapeutic intervention. Eligible are HLA-matched patients who are either carriers of cluster TCRs or are carriers of the known shared tumor antigen.

Owing to novel genetic engineering technologies (CRISPR/Cas9, TALEN, zinc finger nucleases), it more and more becomes feasible to produce allogeneic cellular therapeutic products from healthy donors which are more readily available and at higher numbers than from most patients, and a single product can be used for the treatment of several patients. This is possible because (autologous as well as allogeneic) tsTCRtg-T cells can be genetically engineered to be less immunogenic (e.g. via knock-out of endogenous HLAs in the allogeneic setting), less prone to exhaustion/dysfunction (e.g. per knockout of checkpoint receptors), and less susceptible to induce Graft-versus-host disease (GvHD) or unpredictable crossreactivity due to the knock-out of the endogenous TCRs.

In addition to transducing conventional autologous or allogeneic CD4+ and CD8+ T cells with α/β-tsTCR, it is an option to transduce additional types of adaptive or innate immune cells, like γδ-T cells, NKT cells, and NK cells with the receptors; the genetic engineering technologies mentioned above enable co-transduction of NK cells with tsTCRs and the CD3-signalling domains necessary for the activation of the cells upon TCR-engagement with pMHC.

Therefore, it is highly advantageous to find shared tumor antigens and/or T-cell receptors that are common to more than one individual.

Hence, there is a need to identify shared tumor-specific antigens and/or shared tumor-specific T-cell receptors. This would enable an off-the-shelf treatment for cancer given that the HLAs of the patient are known to match.

In order to achieve this goal, it is necessary to develop a method that identifies such shared tumor-specific TCRs and concomitantly the shared tumor-specific antigen.

A first aspect of the invention relates to a method for identification of a common tumor-specific T cell receptor (TCR).

A second aspect of the invention relates to a method for identification of a common tumor-specific antigen.

A third aspect of the invention relates to an isolated TCR identified by the method according to the first aspect.

A fourth aspect of the invention relates to a nucleic acid sequence encoding the TCR according to the third aspect.

A fifth aspect of the invention relates to an isolated autologous T cell comprising a TCR according to the third aspect, and/or a nucleic acid sequence according to the fourth aspect.

A sixth aspect of the invention relates to the TCR according to the third aspect, the nucleic acid sequence according to the fourth aspect, or the isolated autologous T cell according to the fifth aspect for use in treatment of cancer.

### Terms and definitions

For purposes of interpreting this specification, the following definitions will apply and whenever appropriate, terms used in the singular will also include the plural and vice versa. In the event that any definition set forth below conflicts with any document incorporated herein by reference, the definition set forth shall control.

The terms "comprising," "having," "containing," and "including," and other similar forms, and grammatical equivalents thereof, as used herein, are intended to be equivalent in meaning and to be open ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."

Where a range of values is provided, it is understood that each intervening value, to the tenth of the unit of the lower limit, unless the context clearly dictate otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a," "or," and "the" include plural referents unless the context clearly dictates otherwise.

Unless defined otherwise, all technical and scientific terms used herein have the same meaning as commonly understood by one of ordinary skill in the art (e.g., in cell culture, molecular genetics, nucleic acid chemistry, hybridization techniques and biochemistry). Standard techniques are used for molecular, genetic and biochemical methods (see generally, Sambrook et al., Molecular Cloning: A Laboratory Manual, 4th ed. (2012) Cold Spring Harbor Laboratory Press, Cold Spring Harbor, N.Y. and Ausubel et al., Short Protocols in Molecular Biology (2002) 5th Ed, John Wiley & Sons, Inc.) and chemical methods.

### Sequences

Sequences similar or homologous (e.g., at least about 70% sequence identity) to the sequences disclosed herein are also part of the invention. In some embodiments, the sequence identity at the amino acid level can be about 80%, 85%, 90%, 91 %, 92%, 93%, 94%, 95%, 96%, 97%, 98%, 99% or higher. At the nucleic acid level, the sequence identity can be about 70%, 75%, 80%, 85%, 90%, 91%, 92%, 93%, 94%, 95%, 96%, 97%, 98%, 99% or higher. Alternatively, substantial identity exists when the nucleic acid segments will hybridize under selective hybridization conditions (e.g., very high stringency hybridization conditions), to the complement of the strand. The nucleic acids may be present in whole cells, in a cell lysate, or in a partially purified or substantially pure form.

In the context of the present specification, the terms *sequence identity* and *percentage of sequence identity* refer to a single quantitative parameter representing the result of a sequence comparison determined by comparing two aligned sequences position by position. Methods for alignment of sequences for comparison are well-known in the art. Alignment of sequences for comparison may be conducted by the local homology algorithm of Smith and Waterman, Adv. Appl. Math. 2:482 (1981), by the global alignment algorithm of Needleman and Wunsch, J. Mol. Biol. 48:443 (1970), by the search for similarity method of Pearson and Lipman, Proc. Nat. Acad. Sci. 85:2444 (1988) or by computerized implementations of these algorithms, including, but not limited to: CLUSTAL, GAP, BESTFIT, BLAST, FASTA and TFASTA. Software for performing BLAST analyses is publicly available, e.g., through the National Center for Biotechnology-Information (http://blast.ncbi.nlm.nih.gov/).

One example for comparison of amino acid sequences is the BLASTP algorithm that uses the default settings: Expect threshold: 10; Word size: 3; Max matches in a query range: 0; Matrix: BLOSUM62; Gap Costs: Existence 11, Extension 1; Compositional adjustments: Conditional compositional score matrix adjustment. One such example for comparison of nucleic acid sequences is the BLASTN algorithm that uses the default settings: Expect threshold: 10; Word size: 28; Max matches in a query range: 0; Match/Mismatch Scores: 1.-2; Gap costs: Linear. Unless stated otherwise, sequence identity values provided herein refer to the value obtained using the BLAST suite of programs (Altschul et al., J. Mol. Biol. 215:403-410 (1990)) using the above identified default parameters for protein and nucleic acid comparison, respectively.

Reference to identical sequences without specification of a percentage value implies 100% identical sequences (i.e. the same sequence).

### General Biochemistry: Peptides, Amino Acid Sequences

The term *polypeptide* in the context of the present specification relates to a molecule consisting of 50 or more amino acids that form a linear chain wherein the amino acids are connected by peptide bonds. The amino acid sequence of a polypeptide may represent the amino acid sequence of a whole (as found physiologically) protein or fragments thereof. The term "polypeptides" and "protein" are used interchangeably herein and include proteins and fragments thereof. Polypeptides are disclosed herein as amino acid residue sequences.

The term *peptide* in the context of the present specification relates to a molecule consisting of up to 50 amino acids, in particular 8 to 30 amino acids, more particularly 8 to 15amino acids, that form a linear chain wherein the amino acids are connected by peptide bonds.

Amino acid residue sequences are given from amino to carboxyl terminus. Capital letters for sequence positions refer to L-amino acids in the one-letter code (Stryer, Biochemistry, 3rd ed. p. 21). Lower case letters for amino acid sequence positions refer to the corresponding D- or (2R)-amino acids. Sequences are written left to right in the direction from the amino to the carboxy terminus. In accordance with standard nomenclature, amino acid residue sequences are denominated by either a three letter or a single letter code as indicated as follows: Alanine (Ala, A), Arginine (Arg, R), Asparagine (Asn, N), Aspartic Acid (Asp, D), Cysteine (Cys, C), Glutamine (Gln, Q), Glutamic Acid (Glu, E), Glycine (Gly, G), Histidine (His, H), Isoleucine (Ile, I), Leucine (Leu, L), Lysine (Lys, K), Methionine (Met, M), Phenylalanine (Phe, F), Proline (Pro, P), Serine (Ser, S), Threonine (Thr, T), Tryptophan (Trp, W), Tyrosine (Tyr, Y), and Valine (Val, V).

### General Molecular Biology: Nucleic Acid Sequences, Expression

The term gene refers to a polynucleotide containing at least one open reading frame (ORF) that is capable of encoding a particular polypeptide or protein after being transcribed and translated. A polynucleotide sequence can be used to identify larger fragments or full-length coding sequences of the gene with which they are associated. Methods of isolating larger fragment sequences are known to those of skill in the art.

The terms *gene expression* or *expression,* or alternatively the term *gene product,* may refer to either of, or both of, the processes - and products thereof - of generation of nucleic acids (RNA) or the generation of a peptide or polypeptide, also referred to transcription and translation, respectively, or any of the intermediate processes that regulate the processing of genetic information to yield polypeptide products. The term *gene expression* may also be applied to the transcription and processing of a RNA gene product, for example a regulatory RNA or a structural (e.g. ribosomal) RNA. If an expressed polynucleotide is derived from genomic DNA, expression may include splicing of the mRNA in a eukaryotic cell. Expression may be assayed both on the level of transcription and translation, in other words mRNA and/or protein product.

The term *Nucleotides* in the context of the present specification relates to nucleic acid or nucleic acid analogue building blocks, oligomers of which are capable of forming selective hybrids with RNA or DNA oligomers on the basis of base pairing. The term *nucleotides* in this context includes the classic ribonucleotide building blocks adenosine, guanosine, uridine (and ribosylthymine), cytidine, the classic deoxyribonucleotides deoxyadenosine, deoxyguanosine, thymidine, deoxyuridine and deoxycytidine. It further includes analogues of nucleic acids such as phosphotioates, 2'O-methylphosphothioates, *peptide nucleic acids* (PNA; N-(2-aminoethyl)-glycine units linked by peptide linkage, with the nucleobase attached to the alpha-carbon of the glycine) or *locked nucleic acids* (LNA; 2'O, 4'C methylene bridged RNA building blocks). Wherever reference is made herein to a *hybridizing sequence,* such hybridizing sequence may be composed of any of the above nucleotides, or mixtures thereof.

### T cell biology

The term "CDR3" in the context of the present specification refers to the hypervariable complementarity determining region 3. The size of CDR3 is particularly characterized by the total number of amino acids (AA) and respective nucleotides from the conserved cysteine in the Vβ, or Vα or Vγ or Vδ segment to the position of the conserved phenylalanine in the Jβ or Jα, Jγ or Jδ segment.

The term "T cell activation/exhaustion/differentiation marker" in the context of the present specification refers to a molecule of a T cell, indicative for T cell activation, exhaustion, or differentiation, particularly a molecule on the surface of a T cell.

### (Cancer) Immunotherapy

In the context of the present specification, the term *cancer immunotherapy, biological* or *immunomodulatory therapy* is meant to encompass types of cancer treatment that help the immune system to fight cancer. Non-limiting examples of cancer immunotherapy include immune checkpoint inhibitors and agonists, T cell transfer therapy, cytokines and their recombinant derivatives, adjuvants, and vaccination with small molecules or cells.

The term "tumor sample" in the context of the present specification refers to a sample or a pool of samples obtained from a tumor of a patient. The tumor may also include metastases or a collection of metastases.

The term "non-tumor sample" in the context of the present specification refers to a sample or a pool of samples obtained from tissue in close proximity to the tumor of a patient.

The term "tumor-specific" in the context of the present specification particularly refers to T cells occurring in a particular tumor and particularly exhibiting a preferential distribution in the particular tumor.

The term "HLA" in the context of the present invention refers to the human leukocyte antigen, as a specific subset of the general term major histocompatibility complex (MHC).

HLA supertypes have been defined based on grouping together MHC alleles that share similar binding specificities, i.e. peptides with same or similar so-called anchor amino acid residues (e.g. positions 2 and 9 or 10 in 9- and 10mer peptides). HLA supertypes are further described in Sidney et al. (BMC Immunology 2008, 9:1).

The term *single-cell sequencing* in the context of the present specification refers to a method that allows to identify multiple coding elements of a single cell. This comprises the sequencing of genomic elements such as nuclear or organellar DNA, a transcript thereof or a combination of both. Typically, coding elements of a single cell are linked physically, spatially or by a cell-specific barcode which allows the correct cellular assignment of the elements after sequencing. Particularly, single-cell RNA sequencing (scRNA-seq) is used in order to identify an expression pattern and/or variable sequences of immune receptors. More particularly, scRNA-seq methods allow the identification of mRNAs of at least a 1000 cells in parallel by droplet-based sorting such as 10Xgenomics chromium technology. Other alternatives to characterise cellular expression patterns and/or TCR sequences are methods such as GeoMxTM or CosMxTM by NanoString Technologies Inc. or Visium Spatial Gene Expression by 10X Genomics Inc., or ZipSeq (WO 2019/226631 A1) that are able to associate this information by spatial distribution in samples such as FFPEs (formalin-fixed paraffin embedded).

The term correlation sequencing in the context of the present specification refers to a method that allows a statistical correlation of multiple coding elements of a single cell. This may be achieved by pooling of T-cells or samples comprising pools of T-cells and identifying the transcripts within said pools by sequencing. Those pools that comprise a combination of two or more transcripts are most likely to comprise a common clonotype, which allows to assign this combination by statistical occurrence. Most preferred is the sequencing and correlation of the TCR chain combination of a clonotype.

### Clustering

The clustering of T-cell receptor (TCR) sequences as described in the literature (CDHIT (Limin Fu et al. Bioinformatics. 2012 Dec 1;28(23):3150-2), iSMART (Hongyi Zhang et al. Clin Cancer Res. 2020 Mar 15;26(6):1359-1371), GLIPH (Jacob Glanville et al. Nature. 2017 Jul 6;547(7661):94-98.)) is normally done for one of 2 TCR chains alone. In case of alpha/beta T-cells it is almost always the beta-chain. Another pre-requisite of published TCR clustering approaches is the knowledge about the respective antigenic peptides which are recognized by TCRs via MHC-molecules. In cases of known viral antigenic epitopes these were used as training sets for optimization of the clustering algorithm (GLIPH, iSmart), in case of cancer specific antigenic peptides this is not possible due to lack of known tumor-associated antigens.

The TCRpolyClust method uses a different approach. From the beginning, the clustering algorithm works on TCRs which have an experimentally measured score of being tumor-specific in different patients although the antigenic peptides are unknown. In addition, the algorithm makes explicit use of both chains of the TCR and employs a multi-dimensional scoring to rank the inter-patient TCR clusters by overlaps of HLA-types and expression of various activation/exhaustion/differentiation markers.

### Schematic description of TCRpolyClust

The basic method for providing tumor-specific TCRs (tsTCR) per individual patient is laid out in WO 2017/025564 A1: By quantitative Next-Generation-Sequencing (NGS) of the CDR3 region of T-cells taken from tumor-tissue and at the same time from healthy neighbouring tissue tsTCRs are identified by a clearly enriched frequency in tumor compared to non-tumor tissue: the tumor-specificity ratio is the ratio between clonotype frequencies in tumor versus non-tumor tissue. The method introduced here for characterizing tsTCRs per individual patient comprises the following additional steps.
1) Simultaneous identification of beta- and alpha-chains of the respective tsTCR, preferably by correlation sequencing, more preferably by single-cell VDJ-sequencing, preferably with 10X Genomics chromium technology. As a result, each tsTCR clonotype consists of one beta- and one or two alpha-chain sequences covering the complete CDR3-region and adjacent V- and J-segments. Based on this information the TCR can be fully synthesized.
2) Simultaneous identification of a number of activation/exhaustion/differentiation markers by single-cell gene expression analysis, preferably with 10X Genomics chromium technology. Based on deep sequencing approaches of TIL and single-cell sequencing projects it has become clear that chronic antigen exposure during cancer progression generates tonic TCR signaling which drives T cell exhaustion and suppresses T cell effector function. Exhausted/dysfunctional T cells are "marked" with certain receptors. Inclusion of exhaustion/activation markers in the analysis differentiates bystander clonotypes from tumor-specific clonotypes. Well-known activation/exhaustion markers of CD4+ and CD8+ effector T-cells are PD1, TIGIT, LAG3, TIM3, CTLA4, CD40, CD137 (4-1BB), CD69, or any other genes found significantly expressed in T-cells upon activation by respective antigens. Regarding CD4+ T cells, additional markers are very useful to discriminate tumor-reactive Th1 and Tfh cells from immunosuppressive Th2, Treg, or Th17 cells, which play controversial roles in the context of tumor development. Anti-tumor Th1 and Tfh cells are characterized by expression of effector functions like IFN-y, TNF-α, GZMB, PRF, whereas Th2 cells express IL-4, IL-5, Th17 cells IL-17A, and Tregs immunosuppressive cytokines TGF-β and IL-10, among others. Further, T cell subtypes and/or differentiation states can be differentiated on basis of the expression of certain transcription factors, e.g. FOXP3 for Tregs, TBX21 (TBET), and EOMES for effector T cells, RUNX3 for cells exhibiting cytotoxicity, TOX (exhausted subpopulation of T cells that maintains proliferative and functional capacities), etc.
3) HLA genotyping up to 4-digit resolution is typically performed by analysing patient blood samples with standard methods. In combination with HLA-types, all this information is building up a footprint of the tsTCR repertoire per patient. An example table is given in figure1, the single steps for building the tsTCR foot-print are shown in a flow diagram in figure 2. The tsTCR footprint of a number of patients is the basis of the TCRpolyClust method which is described in the following. A graphical overview is given also in figure 3.
4) A set of different tumor patients is compared via sequence similarities of their TCR CDR3 sequences. The algorithm works in 2 rounds, with round 1 generating a number of seed clusters which are found by grouping all paired TCRs (alpha and beta chain treated as 1 sequence) which on the respective beta- and alpha-chains have a Levenstein distance of maximum 3 (3 mismatches per chain), particularly of maximum 2, more particularly of maximum 1, most particularly of 0. For an efficient pairwise sequence comparison of thousands of TCRs several algorithms are available, e.g. CDHIT, iSMART, BLASTP. Once the seed clustering is completed, a consensus sequence covering the beta-CDR3 amino acid sequences is calculated. In a second round, all consensus sequences of round 1 are subject to clustering with similar parameters as in round 1.
5) The minimum cluster size of an inter-patient cluster is 2, i.e. the cluster must contain alpha-beta paired TCRs from at least two distinct patients. In addition, the median tumor-specificity ratio (i.e. the ratio between clonotype frequencies in tumor compared to non-tumor tissues) has to be > 2 (ideally >3, most preferably >5) and the median clonotype frequency of TCR-beta in tumor tissues has to be at least > 0.005% (particularly > 0.01 %, more particularly > 0.05%, most particularly > 0.1%)
6) The TCR-antigen specificity is strictly dependent on the patient's HLA genotype. An essential condition for a valid inter-patient cluster is therefore a significant overlap in HLA-types within one cluster. At least one HLA-type (A, B, or C) should be found in ≥ 50% of all patients found in that cluster (more preferable in ≥ 60%, most preferable ≥ 80%). In case of a linkage disequilibrium this may apply also to pairs of HLAs up to a complete haplotype.
7) Single-cell RNA sequencing technology (e.g. 10X Genomics) is used to identify both chains of the TCR of a dedicated T-cell and in the same step measures the gene expression profile by scRNAseq. A series of activation/exhaustion markers (e.g. PD1, CTLA4, TIGIT, LAG3, TIM3, etc.), transcription factors (e.g. FOXP3, RUNX3, EOMES, TBET, TOX, etc.), and effector functions (IFN-y, TNF-α, IL-10, IL17, etc.) mentioned above can be measured with respect to their expression frequency so that each single T-cell can be scored with respect to presence of a series or combination of markers. At least one marker should be found in ≥ 50% of all patients found in that cluster (more preferable in ≥ 60%).

The term *common T cell receptor* in the context of the present specification relates to a T cell receptor (TCR) which is present not only in one patient, but which is found in a number of different patients. The common TCR may also be called a shared TCR, as the TCR is shared between patients. This common TCR shares identical or highly similar CDR3 regions among patients, and thus, the common TCR will likely recognize the same antigen. If the common TCR is HLA-dependent, it will likely recognize the same MHC molecule with an identical or almost identical peptide bound to the MHC molecule in different patients. This peptide bound to the MHC molecule shared between patients is therefore a common antigen.

It may be determined in a separate step of the method that all tested patients share at least one HLA-gene (have at least one HLA-gene in common).

The term *common antigen* in the context of the present specification relates to the entity which is recognized by the common TCR. In certain embodiments, this common antigen is a complex comprising an MHC molecule and a peptide bound to the MHC molecule.

The term *essentially identical* in the context of the present specification relates to nucleic acid sequences which are either identical or have an identity of at least 95 %, particularly of at least 97 %, more particularly of at least 98 %, more particularly of at least 99 %, most particularly of more than 99 %.

The term *cross-reactive TCR* in the context of the present specification relates to an alpha/beta TCR that recognises more than one defined peptide sequence presented on an MHC molecule. Preferably, the recognised antigenic peptides differ in at least one amino acid, more preferably two amino acids and are derived from substantially different polypeptide precursors.

The term *frequency* in the context of the present specification relates to a relative abundance of a certain sequence among a plurality of sequences. To determine the frequency of a sequence, the essentially identical sequences are counted and this number is divided by the number of all observed sequences.

The term same *tissue type* in the context of the present specification relates to tissue samples originating from the same tissue. The type of tissue for a metastasis, for example, is the tissue where the metastatic cells originate from, and not the tissue where the metastasis is found inside the body.

The term *gene of the* same *HLA-type* in the context of the present specification relates to the HLA-genes encoding MHC molecules. The same HLA-type herein means that the HLA gene encodes the same variant of an MHC molecule. As there is a large variety of HLA genes in mankind, the HLA repertoire of the tested patients is determined in one embodiment of the method of the invention, and patients sharing at least one gene of the same HLA-type are selected for further analysis.

The term *expressing a sequence in an antigen presenting cell* is understood as follows. The antigen-presenting cell (APC) is transfected or transduced with a nucleic acid sequence encoding a peptide or protein. The nucleic acid sequence is present within a suitable expression vector for transfection or transduction of mammalian cells. Once the nucleic acid sequence is introduced into the APC, the APC expresses the encoded peptide or protein. In certain embodiments, the peptide is presented on an HLA molecule on the cell surface of the APC. In certain embodiments, one or several peptides produced from the protein are presented on an HLA molecule on the cell surface of the APC. Expression of recombinant antigens to be presented on HLA-class I molecules is a well-established procedure that can be achieved by transfection of APCs expressing said class I molecule with nucleic acids coding for said antigens. In certain embodiments, for efficient presentation of recombinant antigens on HLA-class II molecules, respective APCs must express said class II molecule and undergo non-canonical autophagy. Especially non-canonical macroautophaygy is known to re-route intracellularly expressed antigens for processing to be presented on HLA-class II molecules (Münz, Mol Aspects Med. 2021 Jun 16;100987).

The term *activation of T cells* in the context of the present specification relates to a status of a T cell, wherein certain activation markers are expressed. The T cell is specifically activated by the interaction of its TCR with a matching MHC presenting a peptide. The activation can be measured e.g. via IFN-γ secretion of the T cell.

The term *tumor cell line* in the context of the present specification relates to a cell line originating from cancerous tissue. Many tumor cell lines are known in the art (Gandhi et al., Nature. 2019 May; 569 (7757): 503-508.)

A *polymer* of a given group of monomers is a homopolymer (made up of a multiple of the same monomer); a copolymer of a given selection of monomers is a heteropolymer constituted by monomers of at least two of the group.

As used herein, the term *pharmaceutical composition* refers to a compound of the invention, or a pharmaceutically acceptable salt thereof, together with at least one pharmaceutically acceptable carrier. In certain embodiments, the pharmaceutical composition according to the invention is provided in a form suitable for topical, parenteral or injectable administration.

As used herein, the term *pharmaceutically acceptable carrier includes* any solvents, dispersion media, coatings, surfactants, antioxidants, preservatives (for example, antibacterial agents, antifungal agents), isotonic agents, absorption delaying agents, salts, preservatives, drugs, drug stabilizers, binders, excipients, disintegration agents, lubricants, sweetening agents, flavoring agents, dyes, and the like and combinations thereof, as would be known to those skilled in the art (see, for example, Remington: the Science and Practice of Pharmacy, ISBN 0857110624).

As used herein, the term *treating* or *treatment* of any disease or disorder (e.g. cancer) refers in one embodiment, to ameliorating the disease or disorder (e.g. slowing or arresting or reducing the development of the disease or at least one of the clinical symptoms thereof). In another embodiment "treating" or "treatment" refers to alleviating or ameliorating at least one physical parameter including those which may not be discernible by the patient. In yet another embodiment, "treating" or "treatment" refers to modulating the disease or disorder, either physically, (e.g., stabilization of a discernible symptom), physiologically, (e.g., stabilization of a physical parameter), or both. Methods for assessing treatment and/or prevention of disease are generally known in the art, unless specifically described hereinbelow.

### Detailed Description of the Invention

A first aspect of the invention relates to a method for identification of a common tumor-specific T cell receptor (TCR).

The method comprises the steps:
a. obtaining a plurality of tumor-specific TCR sequences from each individual patient of a number n (n>1) of patients via the steps:
   i. in a tumor sequence-based step, determining a frequency of each of a plurality of tumor TCR sequences;
   ii. in a non-tumor sequence-based step, determining a frequency of each of a plurality of non-tumor TCR sequences;
   iii. in a tumor-specific selection step, selecting TCR nucleic acid sequences as tumor-specific TCR sequences if for any particular TCR sequence the frequency of the tumor TCR within the plurality of tumor TCR nucleic acid sequences is higher than the frequency of the non-tumor TCR within the plurality of non-tumor TCR nucleic acid sequences;
b. selecting a common tumor-specific TCR sequence via the steps:
   i. translating tumor-specific TCR nucleic acid sequences into tumor-specific TCR amino acid sequences for a plurality of n patients;
   ii. determining CDR3-regions of said tumor-specific TCR amino acid sequences, yielding tumor-specific CDR3 sequences for each of said n patients;
   iii. aligning the CDR3-regions of said plurality of tumor-specific TCR amino acid sequences of said n patients;
   iv. grouping TCR sequences into one TCR-clonotype cluster if their CDR3-regions differ by no more than 3 amino acids (AAs)/CDR3, particularly by no more than 2 AAs/CDR3, more particularly by no more than 1 AA/CDR3, most particularly do not differ;
   v. in a common TCR selection step, selecting a cluster of TCR sequences as a common tumor-specific TCR sequence if the TCR-clonotype cluster is present in at least two patients.

In certain embodiments, the cluster of TCR sequences is selected as a common tumor-specific TCR sequence if the TCR-clonotype cluster is among the 200 most prevalent tumor-specific TCR sequence, more particularly the 100 most prevalent tumor-specific TCR sequences in at least two patients.

The tumor sequence-based step comprises the steps:
I. providing an isolated tumor sample comprising T cells obtained from said patient;
II. isolating tumor T cells from said isolated tumor sample, yielding isolated tumor T cells;
   isolating a tumor nucleic acid preparation from said isolated tumor T cells;
III. obtaining a plurality of tumor TCR nucleic acid sequences from said tumor nucleic acid preparation;
IV. aligning said plurality of tumor TCR nucleic acid sequences and grouping tumor TCR nucleic acid sequences that are essentially identical into a tumor TCR clonotype group, and counting the tumor TCR nucleic acid sequences in each tumor TCR clonotype group, yielding a number for the tumor TCR nucleic acid sequences in each tumor TCR clonotype group;
V. determining a frequency for each tumor TCR by dividing the number of tumor TCR nucleic acid sequences in each tumor TCR clonotype group by the number of all tumor TCR nucleic acid sequences obtained from the sample

The non-tumor sequence-based step comprises the steps:
I. providing an isolated non-tumor tissue sample comprising T cells obtained from said patient;
II. isolating non-tumor T cells from said isolated non-tumor tissue sample, yielding isolated non-tumor T cells;
   isolating a non-tumor nucleic acid preparation from said isolated non-tumor T cells;
III. obtaining a plurality of non-tumor TCR nucleic acid sequences from said non-tumor nucleic acid preparation;
IV. aligning said plurality of non-tumor TCR nucleic acid sequences and grouping non-tumor TCR nucleic acid sequences into one non-tumor TCR clonotype group that are essentially identical, and counting the non-tumor TCR nucleic acid sequences in each tumor TCR clonotype group, yielding a number for the non-tumor TCR nucleic acid sequences in each non-tumor TCR clonotype group;
V. determining a frequency for each non-tumor TCR by dividing the number of non-tumor TCR nucleic acid sequences in each non-tumor TCR clonotype group by the number of all non-tumor TCR nucleic acid sequences obtained from the sample

In certain embodiments, the method of the first aspect is executed exactly in the order of steps as mentioned above.

In an alternative of the first aspect, the nucleic acid sequences are translated into amino acid sequences before the tumor-specific selection step, and in the tumor-specific selection step, amino acid sequences are compared and selected.

Another alternative of the method of the first aspect may be described as follows:
a. tumor-specific TCR sequences from a first patient are identified via the steps:
   i. sequencing TCR genes from a tumor sample of a first patient;
   ii. sequencing TCR genes from a non-tumor sample of a first patient;
   iii. selecting TCR genes as tumor-specific if these TCR genes are found more often inside the tumor sample than in the non-tumor sample;
b. tumor-specific TCR sequences from a second patient are identified via the same steps i-iii with samples from the second patient, and the method is repeated for a number of n patients;
c. the tumor-specific TCR sequences are compared among the n patients via the steps:
   i. aligning the CDR3 regions of the identified tumor-specific TCR sequences on amino acid level among all patients;
   ii. grouping the TCR sequences into one cluster if the CDR3 region sequences differ by no more than 3 amino acids (AAs)/CDR3, particularly by no more than 2 AAs/CDR3, more particularly by no more than 1 AA/CDR3, most particularly do not differ;
d. a TCR sequence is selected as a common tumor-specific TCR sequence if the cluster is present in at least two patients, particularly in ≥3, ≥4, ≥5, ≥10, ≥20, or ≥30 patients.

In certain embodiments, the tumor sample of the number of n patients is of the same tissue type.

In certain embodiments, said non-tumor tissue sample is of the same tissue type as the tumor sample.

In certain embodiments, the T-cells are sorted after the isolation step II according to their coreceptor CD4 and/or CD8 before obtaining a plurality of non-tumor TCR nucleic acid sequences in step III, thus obtaining either HLA-class I and/or HLA-class II-specific sequences.

In certain embodiments, it is determined that the plurality of patients have at least one gene of the same HLA-type in common.

In certain embodiments, it is determined that the plurality of patients have at least one gene of the same HLA-type in common, wherein the relevant HLA-class can be assigned due to the previous sorting of the T-cells according to CD4+ or CD8+.

In certain embodiments, it is determined that the plurality of patients have a gene of the same HLA-type in common, particularly exactly one gene of the same HLA-type in common, and said common tumor-specific TCR is assigned to an HLA-type specific TCR.

In certain embodiments, said common tumor-specific TCR is assigned to an HLA-type specific TCR via the steps:
a. selecting all patients in whom the common tumor-specific TCR sequence is present;
b. determining which HLA genes are present in said selected patients;
c. assigning the common tumor-specific TCR to an HLA-type specific TCR if a common HLA gene, particularly exactly one common HLA gene, is present in all selected patients.

In certain embodiments, a TCR sequence is selected as a tumor-specific TCR sequence in the tumor-specific selection step (a.iii.) if the frequency of the tumor TCR clonotype group is 2 times higher than the frequency of the non-tumor TCR clonotype group, particularly 3 times higher, more particularly 5 times higher, even more particularly 10 times higher than the frequency of the non-tumor TCR clonotype group.

In certain embodiments, the number of patients n is 2 to 100, particularly n is 10 to 50, more particularly n is 20 to 30.

In certain embodiments, the common TCR selection step (step b.v) additionally comprises a measurement of T cell activation/exhaustion/differentiation markers, particularly a marker selected from PDCD1 (PD1), TIGIT, LAG3, HAVCR2 (TIM3), CTLA4, IFNG, TNF, GZMB, TNFRSF9 (CD137, 4-1BB), CD45 (CD45RA/RO), CD69, LAMP1 (CD107a), TBX21 (T-BET), TCF7 (TCF-1), EOMES, TOX, and RUNX3, wherein a TCR sequence is selected as a common tumor-specific TCR sequence if the T cells carrying the TCR express one or more T cell activation/exhaustion/differentiation markers or combinations thereof.

A second aspect of the invention relates to a method for identification of a common tumor-specific antigen.

The method comprises the steps:
a. identification of a common tumor-specific TCR according to the first aspect from a number n (n>1) of patients;
b. obtaining a plurality of tumor-specific polypeptides via the steps:
   (1) obtaining a plurality of tumor-specific mRNA sequences from each patient from said patients via the steps:
      i. in an mRNA tumor sequence-based step, determining a plurality of tumor mRNA sequences;
      ii. in an mRNA non-tumor sequence-based step, determining a plurality of non-tumor mRNA sequences;
      iii. in an mRNA tumor-specific selection step, selecting tumor-specific mRNA sequences;
   (2) selecting a plurality of tumor-specific polypeptides via the steps:
      i. translating said plurality of tumor-specific RNA sequences into a plurality of tumor-specific amino acid sequences;
      ii. aligning the plurality of tumor-specific amino acid sequences from the plurality of n patients;
      iii. grouping amino acid sequences into one polypeptide cluster if their amino acid sequences have a sequence identity of ≥80%, ≥85%, ≥90%, ≥92%, ≥94%, ≥96%, ≥98%, or ≥99%;
      iv. selecting a plurality of polypeptide clusters of amino acid sequences as tumor-specific polypeptides if the tumor-specific amino acid sequence is present in all of the n patients (all patients from whom the common tumor-specific TCR was selected from);
c. for each member of the plurality of tumor-specific polypeptides, expressing said member of the plurality of tumor-specific polypeptides in an antigen-presenting cell sharing an HLA-gene with the patients from whom the common tumor-specific TCR was selected from;
d. detecting for each antigen-presenting cell whether the antigen-presenting cell is able to activate a T cell expressing said common tumor-specific TCR;
e. selecting a tumor-specific polypeptide as a common tumor-specific antigen if the antigen-presenting cell expressing said tumor-specific polypeptide is able to activate said T cell expressing said common tumor-specific TCR.

The mRNA tumor sequence-based step comprises the steps:
I. isolating a tumor RNA preparation from a tumor sample from said patient;
II. obtaining a plurality of tumor mRNA sequences from said tumor RNA preparation.

The mRNA non-tumor sequence-based step comprises the steps:
I. isolating a non-tumor RNA preparation from said non-tumor tissue sample from said patient;
II. obtaining a plurality of non-tumor mRNA sequences from said tumor RNA preparation.

The mRNA tumor-specific selection step comprises the steps:
I. aligning the plurality of tumor mRNA sequences and the plurality of non-tumor mRNA sequences;
II. selecting mRNA sequences which are present in the tumor sample and absent (not detectable) in the non-tumor sample as tumor-specific RNA sequences.

In certain embodiments, additionally a peptide presented on an HLA molecule on the antigen-presenting cell expressing said common tumor-specific antigen is isolated from the HLA molecule and characterized via mass-spectrometry (Freudenmann et al., Immunology. 2018 Jul;154(3):331-345.).

In certain embodiments, additionally
a. the antigen found by the method of the second aspect is fragmented into peptides;
b. each peptide is loaded on an HLA molecule on an antigen-presenting cell;
c. for each antigen-presenting cell it is determined whether the antigen-presenting cell is able to activate a T cell expressing the common tumor-specific TCR found by the method according to the first aspect.

In certain embodiments, said tumor sample and said non-tumor tissue sample originate from the same tissue sample and wherein isolating a tumor RNA preparation from said tissue sample is performed separately from single tumor cells and non-tumor cells obtained from said tissue sample.

Single cell sequencing is a convenient way to identify tumor-specific RNA. Since the tumor always represents a mixture of cancer cells and normal cells, the reference RNA of the normal tissue is automatically included. However, cells from the neighboring tissue can also be used as a reference.

An alternative of the second aspect relates to a method for identification of a common tumor-specific antigen, the method comprising the steps:
a. identification of a common tumor-specific TCR according to the first aspect from a number n (n>1) of patients;
b. contacting a T cell expressing said common tumor-specific TCR with a tumor cell, wherein the tumor cell is derived from a tumor cell line (expressing an HLA-gene shared between the patients from whom the common tumor-specific TCR was selected from) and detecting whether the tumor cell is able to activate the T cell yielding a tumor-cell-line derived cell expressing the common tumor-specific antigen;
c. optionally repeating step b with a different tumor cell line;
d. preparing a cDNA library from the tumor-cell-line derived cell expressing the common tumor-specific antigen;
e. for each member of the cDNA library, expressing said member in an antigen-presenting cell having an HLA-gene shared between the patients from whom the common tumor-specific TCR was selected from;
f. detecting for each antigen-presenting cell whether the antigen-presenting cell is able to activate a T cell expressing said common tumor-specific TCR;
g. selecting a cDNA as a common tumor-specific antigen if the antigen-presenting cell expressing said cDNA is able to activate said T cell expressing said common tumor-specific TCR.

Alternatively, it is possible to take the tumor cell line and knock out individual sections or genes. Afterwards, one has to identify those clones which are no longer recognized. Approaches with transposon (random) or CRISPR-Cas (targeted) are conceivable.

Another alternative of the method of the second aspect may be described as follows:
a. a common tumor-specific TCR sequence is identified via the first aspect;
b. common tumor-specific amino acid sequences are identified via the steps:
   i. sequencing the mRNA repertoire of the tumor sample of the first patient;
   ii. sequencing the mRNA repertoire of the non-tumor sample of the first patient;
   iii. selecting mRNA sequences as tumor-specific in the first patient if the mRNA sequence is present in the tumor sample and absent from the non-tumor sample;
   iv. repeating steps i-iii for all n patients;
   v. translating the tumor-specific mRNA sequences of all patients into amino acid sequences and aligning the tumor-specific amino acid sequences;
   vi. grouping amino acid sequences into one cluster if their amino acid sequences have a sequence identity of ≥80%, ≥85%, ≥90%, ≥92%, ≥94%, ≥96%, ≥98%, or ≥99%;
   vii. selecting sequences as common tumor-specific amino acid sequences if the respective cluster is present in all tested patients;
c. the reactivity of the common tumor-specific TCR against the common tumor-specific amino acid sequences is evaluated via the steps:
   i. introducing each common tumor-specific amino acid sequence into one antigen-presenting cell;
   ii. contacting a T cell expressing the common tumor-specific TCR with all the antigen-presenting cells of step i separately, and measuring IFNy release of the T cell;
d. the amino acid sequence is selected as a common tumor-specific antigen, for which there was a detectable IFNy release of the T cell in the previous step.

A third aspect of the invention relates to an isolated TCR identified by the method according to the first aspect.

In certain embodiments, the TCR comprises a CDR3 alpha sequence and a CDR3 beta sequence, wherein the CDR3 alpha sequence and the CDR3 beta sequence are identical to the sequences given below, or with one or two amino acid substitutions per CDR3 sequence,
wherein
a. for group a (Cluster 0), the CDR3 alpha sequence is selected from the group of sequences comprising SEQ ID NO: 6, 1, 59, and the CDR3 beta sequence is selected from the group of sequences comprising SEQ ID NO: 38, 41, 20, or
b. for group b (Cluster 1), the CDR3 alpha sequence is selected from the group of sequences comprising SEQ ID NO: 11, 12, 18, 26, 55, 58, and the CDR3 beta sequence is selected from the group of sequences comprising SEQ ID NO: 47, 72, 73, or
c. for group c (Cluster 31), the CDR3 alpha sequence is selected from the group of sequences comprising SEQ ID NO: 62, 63, 71, and the CDR3 beta sequence is selected from the sequence SEQ ID NO: 28, or
d. for group d (Cluster 2), the CDR3 alpha sequence is selected from the group of sequences comprising SEQ ID NO: 8, 51, and the CDR3 beta sequence is selected from the group of sequences comprising SEQ ID NO: 19, 45, 60, or
e. for group e (Cluster 3), the CDR3 alpha sequence is selected from the group of sequences comprising SEQ ID NO: 2, 21, 24, 32, and the CDR3 beta sequence is selected from the group of sequences comprising SEQ ID NO: 15, 27, 31, or
f. for group f (Cluster 4), the CDR3 alpha sequence is selected from the group of sequences comprising SEQ ID NO: 29, 52, and the CDR3 beta sequence is selected from the sequence SEQ ID NO: 9, or
g. for group g (Cluster 5), the CDR3 alpha sequence is selected from the group of sequences comprising SEQ ID NO: 13, 65, and the CDR3 beta sequence is selected from the sequence SEQ ID NO: 61, or
h. for group h (Cluster 6), the CDR3 alpha sequence is selected from the group of sequences comprising SEQ ID NO: 5, 25, 46, and the CDR3 beta sequence is selected from the group of sequences comprising SEQ ID NO: 7, 10, 17, 42, 44,
   particularly wherein the CRD3 alpha sequence and the CDR3 beta sequence are identified in the same row of tables 1-8,
   particularly wherein said substitutions are selected according to the substitution rules given below,
   wherein the substitution rules are:
      - glycine (G) and alanine (A) are interchangeable; valine (V), leucine (L), and isoleucine (I) are interchangeable, A and V are interchangeable;
      - tryptophan (W) and phenylalanine (F) are interchangeable, tyrosine (Y) and F are interchangeable;
      - serine (S) and threonine (T) are interchangeable;
      - aspartic acid (D) and glutamic acid (E) are interchangeable
      - asparagine (N) and glutamine (Q) are interchangeable; N and S are interchangeable; N and D are interchangeable; E and Q are interchangeable;
      - methionine (M) and Q are interchangeable;
      - cysteine (C), A and S are interchangeable;
      - proline (P), G and A are interchangeable;
      - arginine (R) and lysine (K) are interchangeable.

A group of CDR3 sequences may also be called a cluster.

In certain embodiments, the CDR3 sequences are selected from the groups a, b, f, g, and h.

In certain embodiments, the TCR additionally comprises a variable (V) alpha sequence, a joining-constant (JC) alpha sequence, a V beta sequence, and a JC beta sequence or a sequence with ≥80%, ≥85%, ≥90%, ≥92%, ≥94%, ≥96%, ≥98%, or ≥99% sequence identity to said sequences, wherein the complete TCR sequence retains its biological activity,
wherein
a. for group a, the V alpha sequence is SEQ ID NO: 67, the JC alpha sequence is SEQ ID NO: 4, the V beta sequence is SEQ ID NO: 23, and the JC beta sequence is SEQ ID NO: 33, or
b. for group b, the V alpha sequence is SEQ ID NO: 3, the JC alpha sequence is SEQ ID NO: 53, the V beta sequence is SEQ ID NO: 64, and the JC beta sequence is SEQ ID NO: 56, or
c. for group c, the V alpha sequence is SEQ ID NO: 54, the JC alpha sequence is SEQ ID NO: 48, the V beta sequence is SEQ ID NO: 43, and the JC beta sequence is SEQ ID NO: 37, or
d. for group d, the V alpha sequence is SEQ ID NO: 68, the JC alpha sequence is SEQ ID NO: 70, the V beta sequence is SEQ ID NO: 30, and the JC beta sequence is SEQ ID NO: 37, or
e. for group e, the V alpha sequence is SEQ ID NO: 57, the JC alpha sequence is SEQ ID NO: 4, the V beta sequence is SEQ ID NO: 16, and the JC beta sequence is SEQ ID NO: 22, or
f. for group f, the V alpha sequence is SEQ ID NO: 49, the JC alpha sequence is SEQ ID NO: 66, the V beta sequence is SEQ ID NO: 39, and the JC beta sequence is SEQ ID NO: 34, or
g. for group g, the V alpha sequence is SEQ ID NO: 35, the JC alpha sequence is SEQ ID NO: 40, the V beta sequence is SEQ ID NO: 43, and the JC beta sequence is SEQ ID NO: 69, or
h. for group h, the V alpha sequence is SEQ ID NO: 14, the JC alpha sequence is SEQ ID NO: 50, the V beta sequence is SEQ ID NO: 36, and the JC beta sequence is SEQ ID NO: 56.

The biological activity of a TCR is determined via the activation of the T cell comprising the TCR by a tumor cell or an APC. If the TCR is still able to recognize an APC presenting the HLA-peptide complex for which the TCR is specific, then the TCR retains its biological activity, even in cases with a deviating sequence.

A fourth aspect of the invention relates to a nucleic acid sequence encoding the TCR according to the third aspect.

A fifth aspect of the invention relates to an isolated autologous T cell comprising a TCR according to the third aspect, and/or a nucleic acid sequence according to the fourth aspect.

In certain embodiments, the isolated autologous T cell is a recombinant T cell recombinantly expressing said TCR.

A sixth aspect of the invention relates to the TCR according to the third aspect, the nucleic acid sequence according to the fourth aspect, or the isolated autologous T cell according to the fifth aspect for use in treatment of cancer.

The TCR according to the third aspect, the nucleic acid sequence according to the fourth aspect, or the isolated autologous T cell according to the fifth aspect for use in treatment of cancer in patients having the same HLA-type as the HLA-type specific TCR determined via the method of the first aspect.

The TCR according to the third aspect, the nucleic acid sequence according to the fourth aspect, or the isolated autologous T cell according to the fifth aspect for use in patients with the following HLA-type:
a. HLA-B*08:01 and/or HLA-C*07:01 for group a; or
b. HLA-A*02:01-supertype (HLA-A*02:01/68:02) for group b; or
c. HLA-A*02:01-supertype (HLA-A*02:01/02:35/02:05) and/or HLA-C*07:01/07:04 for group c; or
d. HLA-A*01:01 and/or HLA-A*02:01 for group d; or
e. HLA-A*02:01 and/or the HLA-A*01-supertype (A*01 :01/68:01) for group e; or
f. HLA-C*07:01 for group f; or
g. HLA-A*01:01 and/or HLA-A*02:01 and/or HLA-C*02:02 for group g; or
h. HLA-B*15:01 for group h.

### Medical treatment, Dosage Forms and Salts

Similarly, within the scope of the present invention is a method or treating cancer in a patient in need thereof, comprising administering to the patient a the TCR according to the third aspect, the nucleic acid sequence according to the fourth aspect, or the isolated autologous T cell according to the fifth aspect.

Similarly, a dosage form for the prevention or treatment of cancer is provided, comprising a the TCR according to the third aspect, the nucleic acid sequence according to the fourth aspect, or the isolated autologous T cell according to the fifth aspect.

### Pharmaceutical Compositions and Administration

Another aspect of the invention relates to a pharmaceutical composition comprising the TCR according to the third aspect, the nucleic acid sequence according to the fourth aspect, or the isolated autologous T cell according to the fifth aspect.

In certain embodiments of the invention, the compound of the present invention is typically formulated into pharmaceutical dosage forms to provide an easily controllable dosage of the drug and to give the patient an elegant and easily manageable product.

The pharmaceutical composition can be formulated for parenteral administration, for example by i.v. infusion.

### Method of Manufacture and Method of Treatment according to the invention

The invention further encompasses, as an additional aspect, the use of the TCR according to the third aspect, the nucleic acid sequence according to the fourth aspect, or the isolated autologous T cell according to the fifth aspect, as specified in detail above, for use in a method of manufacture of a medicament for the treatment or prevention of cancer.

Similarly, the invention encompasses methods of treatment of a patient having been diagnosed with a disease associated with cancer. This method entails administering to the patient the TCR according to the third aspect, the nucleic acid sequence according to the fourth aspect, or the isolated autologous T cell according to the fifth aspect.

The specification further encompasses the following items:

### Items:

1. A method for identification of a common tumor-specific T cell receptor (TCR), the method comprising the steps:
   a. obtaining a plurality of tumor-specific TCR sequences from each individual patient of a number n (n>1) of patients via the steps:
      i. in a tumor sequence-based step, determining a frequency of each of a plurality of tumor TCR sequences via the steps:
         I. providing an isolated tumor sample obtained from said patient;
         II. isolating tumor T cells from said tumor sample;
         III. obtaining a plurality of tumor TCR nucleic acid sequences;
         IV. grouping tumor TCR nucleic acid sequences that are essentially identical into a tumor TCR clonotype group, and counting the tumor TCR nucleic acid sequences in each tumor TCR clonotype group;
         V. determining a frequency for each tumor TCR by dividing the number of tumor TCR nucleic acid sequences in each tumor TCR clonotype group by the number of all tumor TCR nucleic acid sequences;
      ii. in a non-tumor sequence-based step, determining a frequency of each of a plurality of non-tumor TCR sequences via the steps:
         I. providing an isolated non-tumor tissue sample obtained from said patient;
         II. isolating non-tumor T cells from said non-tumor tissue sample;
         III. obtaining a plurality of non-tumor TCR nucleic acid sequences;
         IV. grouping non-tumor TCR nucleic acid sequences into one non-tumor TCR clonotype group that are essentially identical, and counting the non-tumor TCR nucleic acid sequences in each tumor TCR clonotype group;
         V. determining a frequency for each non-tumor TCR by dividing the number of non-tumor TCR nucleic acid sequences in each non-tumor TCR clonotype group by the number of all non-tumor TCR nucleic acid sequences;
      iii. in a tumor-specific selection step, selecting TCR nucleic acid sequences as tumor-specific TCR sequences if the frequency of the tumor TCR is higher than the frequency of the non-tumor TCR;
   b. selecting a common tumor-specific TCR sequence via the steps:
      i. translating tumor-specific TCR nucleic acid sequences into tumor-specific TCR amino acid sequences;
      ii. determining CDR3-regions of said tumor-specific TCR amino acid sequences of said n patients;
      iii. aligning the CDR3-regions of said plurality of tumor-specific TCR amino acid sequences of said n patients;
      iv. grouping TCR sequences into one TCR-clonotype cluster if their CDR3-regions differ by no more than 3 amino acids (AAs)/CDR3, particularly by no more than 2 AAs/CDR3, more particularly by no more than 1 AA/CDR3, most particularly do not differ;
      v. in a common TCR selection step, selecting a cluster of TCR sequences as a common tumor-specific TCR sequence if the TCR-clonotype cluster is present in at least two patients.
2. The method according to item 1, wherein the tumor sample of the number of n patients is of the same tissue type.
3. The method according to any one of the preceding items, wherein said non-tumor tissue sample is of the same tissue type as the tumor sample.
4. The method according to any one of the preceding items, wherein it is determined that the plurality of patients have at least one gene of the same HLA-type in common.
5. The method according to item 4, wherein it is determined that the plurality of patients have a gene of the same HLA-type in common, particularly exactly one gene of the same HLA-type in common, and said common tumor-specific TCR is assigned to an HLA-type specific TCR.
6. The method according to any one of the preceding items, wherein said common tumor-specific TCR is assigned to an HLA-type specific TCR via the steps:
   a. selecting all patients in whom the common tumor-specific TCR sequence is present;
   b. determining which HLA genes are present in said selected patients;
   c. assigning the common tumor-specific TCR to an HLA-type specific TCR if a common HLA gene, particularly exactly one common HLA gene, is present in all selected patients.
7. The method according to any one of the preceding items, wherein a TCR sequence is selected as a tumor-specific TCR sequence in the tumor-specific selection step (a.iii.) if the frequency of the tumor TCR clonotype group is 2 times higher than the frequency of the non-tumor TCR clonotype group, particularly 3 times higher, more particularly 5 times higher, even more particularly 10 times higher than the frequency of the non-tumor TCR clonotype group.
8. The method according to any one of the preceding items, wherein the number of patients n is 2 to 100, particularly n is 10 to 50, more particularly n is 20 to 30.
9. The method according to any one of the preceding items, wherein the common TCR selection step (step b.v) additionally comprises a measurement of T cell activation/exhaustion/differentiation markers, particularly a marker selected from PDCD1 (PD1), TIGIT, LAG3, HAVCR2 (TIM3), CTLA4, IFNG, TNF, GZMB, TNFRSF9 (CD137, 4-1BB), CD45 (CD45RA/RO), CD69, LAMP1 (CD107a), TBX21 (T-BET), TCF7 (TCF-1), EOMES, TOX, and RUNX3, wherein a TCR sequence is selected as a common tumor-specific TCR sequence if the T cells carrying the TCR express one or more T cell activation/exhaustion/differentiation markers or combinations thereof.
10. A method for identification of a common tumor-specific antigen, the method comprising the steps:
   a. identification of a common tumor-specific TCR according to any one of the preceding items from a number n (n>1) of patients;
   b. obtaining a plurality of tumor-specific polypeptides via the steps:
      (1) obtaining a plurality of tumor-specific mRNA sequences from each patient from said patients via the steps:
         i. in an mRNA tumor sequence-based step, determining a plurality of tumor mRNA sequences via the steps:
            I. isolating a tumor RNA preparation from a tumor sample from said patient;
            II. obtaining a plurality of tumor mRNA sequences from said tumor RNA preparation;
         ii. in an mRNA non-tumor sequence-based step, determining a plurality of non-tumor mRNA sequences via the steps:
            I. isolating a non-tumor RNA preparation from said non-tumor tissue sample from said patient;
            II. obtaining a plurality of non-tumor mRNA sequences from said tumor RNA preparation;
         iii. in an mRNA tumor-specific selection step, selecting tumor-specific mRNA sequences via the steps:
            I. aligning the plurality of tumor mRNA sequences and the plurality of non-tumor mRNA sequences;
            II. selecting mRNA sequences which are present in the tumor sample and absent in the non-tumor sample as tumor-specific RNA sequences;
      (2) selecting a plurality of tumor-specific polypeptides via the steps:
         i. translating said plurality of tumor-specific RNA sequences into a plurality of tumor-specific amino acid sequences;
         ii. aligning the plurality of tumor-specific amino acid sequences from the plurality of n patients;
         iii. grouping amino acid sequences into one polypeptide cluster if their amino acid sequences have a sequence identity of ≥80%, ≥85%, ≥90%, ≥92%, ≥94%, ≥96%, ≥98%, or ≥99%;
         iv. selecting a plurality of polypeptide clusters of amino acid sequences as tumor-specific polypeptides if the tumor-specific amino acid sequence is present in all of the n patients;
   c. for each member of the plurality of tumor-specific polypeptides, expressing said member in an antigen-presenting cell;
   d. detecting for each antigen-presenting cell whether the antigen-presenting cell is able to activate a T cell expressing said common tumor-specific TCR;
   e. selecting a tumor-specific polypeptide as a common tumor-specific antigen if the antigen-presenting cell expressing said tumor-specific polypeptide is able to activate said T cell expressing said common tumor-specific TCR.
11. The method according to item 10, wherein additionally a peptide presented on an HLA molecule on the antigen-presenting cell expressing said common tumor-specific antigen is isolated and characterized via mass-spectrometry.
12. The method according to item 10, wherein subsequently
   a. the antigen found by the method of item 10 is fragmented into peptides;
   b. each peptide is loaded on an HLA molecule on an antigen-presenting cell;
   c. for each antigen-presenting cell it is determined whether the antigen-presenting cell is able to activate a T cell expressing the common tumor-specific TCR.
13. The method according to any one of the preceding items 10, 11, or 12, wherein said tumor sample and said non-tumor tissue sample originate from the same tissue sample and wherein isolating a tumor RNA preparation from said tissue sample is performed separately from single tumor cells and non-tumor cells obtained from said tissue sample.
14. A method for identification of a common tumor-specific antigen, the method comprising the steps:
   a. identification of a common tumor-specific TCR according to any one of the preceding items from a number n (n>1) of patients;
   b. contacting a T cell expressing said common tumor-specific TCR with a tumor cell, wherein the tumor cell is derived from a tumor cell line and detecting
      whether the tumor cell is able to activate the T cell yielding a tumor-cell-line derived cell expressing the common tumor-specific antigen;
   c. optionally repeating step b with a different tumor cell line;
   d. preparing a cDNA library from the tumor-cell-line derived cell expressing the common tumor-specific antigen;
   e. for each member of the cDNA library, expressing said member in an antigen-presenting cell;
   f. detecting for each antigen-presenting cell whether the antigen-presenting cell is able to activate a T cell expressing said common tumor-specific TCR;
   g. selecting a cDNA as a common tumor-specific antigen if the antigen-presenting cell expressing said cDNA is able to activate said T cell expressing said common tumor-specific TCR.
15. An isolated TCR identified by the method according to any one of items 1 to 9.
16. The isolated TCR according to item 15, wherein the TCR comprises a CDR3 alpha sequence and a CDR3 beta sequence, wherein the CDR3 alpha sequence and the CDR3 beta sequence are identical to the sequences given below, or with one or two amino acid substitutions per CDR3 sequence,
   wherein
   a. for group a, the CDR3 alpha sequence is selected from the group of sequences comprising SEQ ID NO 6, SEQ ID NO 1, SEQ ID NO 59, and the CDR3 beta sequence is selected from the group of sequences comprising SEQ ID NO 38, SEQ ID NO 41, SEQ ID NO 20, or
   b. for group b, the CDR3 alpha sequence is selected from the group of sequences comprising SEQ ID NO 11, SEQ ID NO 12, SEQ ID NO 18, SEQ ID NO 26, SEQ ID NO 55, SEQ ID NO 58, and the CDR3 beta sequence is selected from the group of sequences comprising SEQ ID NO 47, SEQ ID NO 72, SEQ ID NO 73, or
   c. for group c, the CDR3 alpha sequence is selected from the group of sequences comprising SEQ ID NO 62, SEQ ID NO 63, SEQ ID NO 71, and the CDR3 beta sequence is selected from the sequence SEQ ID NO 28, or
   d. for group d, the CDR3 alpha sequence is selected from the group of sequences comprising SEQ ID NO 8, SEQ ID NO 51, and the CDR3 beta sequence is selected from the group of sequences comprising SEQ ID NO 19, SEQ ID NO 45, SEQ ID NO 60, or
   e. for group e, the CDR3 alpha sequence is selected from the group of sequences comprising SEQ ID NO 2, SEQ ID NO 21, SEQ ID NO 24, SEQ ID NO 32, and the CDR3 beta sequence is selected from the group of sequences comprising SEQ ID NO 15, SEQ ID NO 27, SEQ ID NO 31, or
   f. for group f, the CDR3 alpha sequence is selected from the group of sequences comprising SEQ ID NO 29, SEQ ID NO 52, and the CDR3 beta sequence is selected from the sequence SEQ ID NO 9, or
   g. for group g, the CDR3 alpha sequence is selected from the group of sequences comprising SEQ ID NO 13, SEQ ID NO 65, and the CDR3 beta sequence is selected from the sequence SEQ ID NO 61, or
   h. for group h, the CDR3 alpha sequence is selected from the group of sequences comprising SEQ ID NO 5, SEQ ID NO 25, SEQ ID NO 46, and the CDR3 beta sequence is selected from the group of sequences comprising SEQ ID NO 7, SEQ ID NO 10, SEQ ID NO 17, SEQ ID NO 42, SEQ ID NO 44,
      particularly wherein the CRD3 alpha sequence and the CDR3 beta sequence are identified in the same row of tables 1-8,
      particularly wherein said substitutions are selected according to the substitution rules given below,
   wherein the substitution rules are:
   - glycine (G) and alanine (A) are interchangeable; valine (V), leucine (L), and isoleucine (I) are interchangeable, A and V are interchangeable;
   - tryptophan (W) and phenylalanine (F) are interchangeable, tyrosine (Y) and F are interchangeable;
   - serine (S) and threonine (T) are interchangeable;
   - aspartic acid (D) and glutamic acid (E) are interchangeable
   - asparagine (N) and glutamine (Q) are interchangeable; N and S are interchangeable; N and D are interchangeable; E and Q are interchangeable;
   - methionine (M) and Q are interchangeable;
   - cysteine (C), A and S are interchangeable;
   - proline (P), G and A are interchangeable;
   - arginine (R) and lysine (K) are interchangeable.
17. The isolated TCR according to item 16, wherein the CDR3 sequences are selected from the groups a, b, f, g, and h.
18. The isolated TCR according to item 16 or 17, wherein the TCR additionally comprises a variable (V) alpha sequence, a joining-constant (JC) alpha sequence, a V beta sequence, and a JC beta sequence or a sequence with ≥80%, ≥85%, ≥90%, ≥92%, ≥94%, ≥96%, ≥98%, or ≥99% sequence identity to said sequences,
   wherein
   a. for group a, the V alpha sequence is SEQ ID NO: 67, the JC alpha sequence is SEQ ID NO: 4, the V beta sequence is SEQ ID NO: 23, and the JC beta sequence is SEQ ID NO: 33, or
   b. for group b, the V alpha sequence is SEQ ID NO: 3, the JC alpha sequence is SEQ ID NO: 53, the V beta sequence is SEQ ID NO: 64, and the JC beta sequence is SEQ ID NO: 56, or
   c. for group c, the V alpha sequence is SEQ ID NO: 54, the JC alpha sequence is SEQ ID NO: 48, the V beta sequence is SEQ ID NO: 43, and the JC beta sequence is SEQ ID NO: 37, or
   d. for group d, the V alpha sequence is SEQ ID NO: 68, the JC alpha sequence is SEQ ID NO: 70, the V beta sequence is SEQ ID NO: 30, and the JC beta sequence is SEQ ID NO: 37, or
   e. for group e, the V alpha sequence is SEQ ID NO: 57, the JC alpha sequence is SEQ ID NO: 4, the V beta sequence is SEQ ID NO: 16, and the JC beta sequence is SEQ ID NO: 22, or
   f. for group f, the V alpha sequence is SEQ ID NO: 49, the JC alpha sequence is SEQ ID NO: 66, the V beta sequence is SEQ ID NO: 39, and the JC beta sequence is SEQ ID NO: 34, or
   g. for group g, the V alpha sequence is SEQ ID NO: 35, the JC alpha sequence is SEQ ID NO: 40, the V beta sequence is SEQ ID NO: 43, and the JC beta sequence is SEQ ID NO: 69, or
   h. for group h, the V alpha sequence is SEQ ID NO: 14, the JC alpha sequence is SEQ ID NO: 50, the V beta sequence is SEQ ID NO: 36, and the JC beta sequence is SEQ ID NO: 56.
19. A nucleic acid sequence encoding the TCR according to any one of items 15 to 18.
20. An isolated autologous T cell comprising a TCR according to any one of items 15 to 18, and/or a nucleic acid sequence according to item 19.
21. The isolated autologous T cell according to item 20, wherein the isolated autologous T cell is a recombinant T cell.
22. The TCR according to any one of items 15 to 18, the nucleic acid sequence according to item 19, or the isolated autologous T cell according to items 20 or 21 for use in treatment of cancer.
23. The TCR according to any one of items 15 to 18, the nucleic acid sequence according to item 19, or the isolated autologous T cell according to items 20 or 21 for use in treatment of cancer in patients having the same HLA-type as the HLA-type specific TCR determined via the method according to item 5 or 6.
24. The TCR according to any one of items 15 to 18, the nucleic acid sequence according to item 19, or the isolated autologous T cell according to items 20 or 21 for use according to item 23 in patients with the following HLA-type:
   a. HLA-B*08:01 and/or HLA-C*07:01 for group a; or
   b. HLA-A*02:01-supertype (HLA-A*02:01/68:02) for group b; or
   c. HLA-A*02:01-supertype (HLA-A*02:01/02:35/02:05) and/or HLA-C*07:01/07:04 for group c; or
   d. HLA-A*01:01 and/or HLA-A*02:01 for group d; or
   e. HLA-A*02:01 and/or the HLA-A*01-supertype (A*01:01/68:01) for group e; or
   f. HLA-C*07:01 for group f; or
   g. HLA-A*01:01 and/or HLA-A*02:01 and/or HLA-C*02:02 for group g; or
   h. HLA-B*15:01 for group h.

The invention is further illustrated by the following examples and figures, from which further embodiments and advantages can be drawn. These examples are meant to illustrate the invention but not to limit its scope.

### Description of the Figures

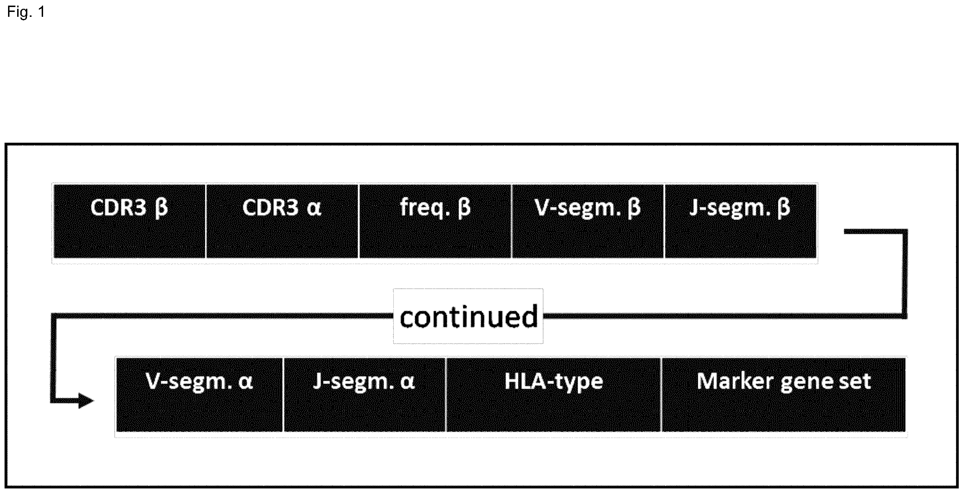

Fig.1: Schema of tsTCR foot print table. From left to right the tumour specific TCR footprint table comprises the following elements per TCR clonotype: CDR3 β amino acid sequence, CDR3 α amino acid sequence, frequency of CDR3 β sequence in percent, V-segment ID of β chain, J-segment ID of β chain, V-segment ID of α chain, J-segment ID of α chain, HLA-type(s) in 4-digit resolution (class I or II), a set of marker genes in several columns with respective expression rates per clonotype.

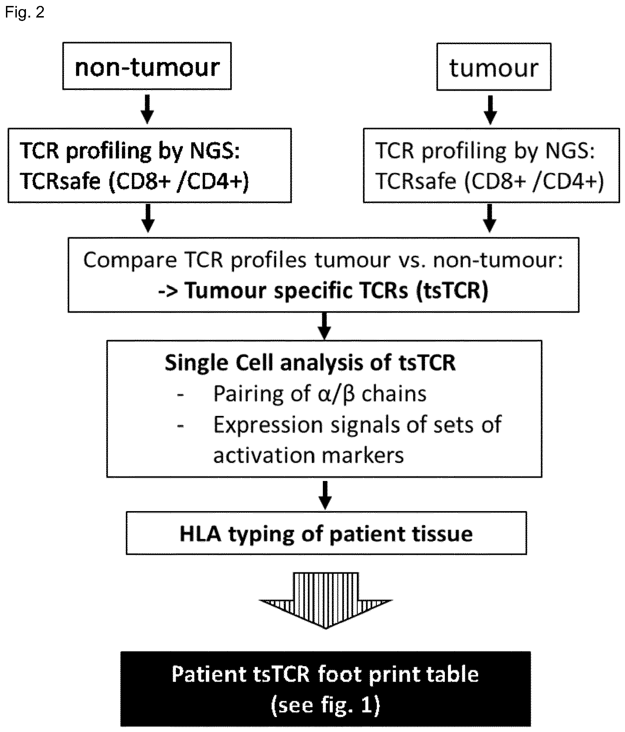

Fig. 2: TCR clustering schema, part I. These are the steps for a TCR repertoire analysis in tumour and non-tumour condensed finally into a tumour-specific TCR foot print table

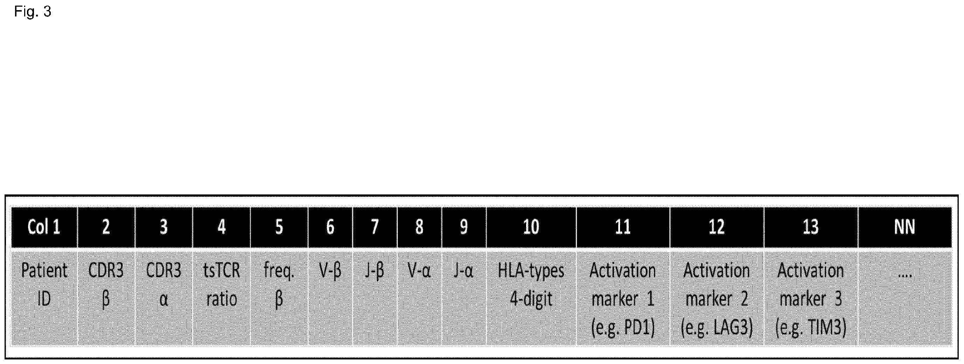

Fig. 3: TCR clustering schema, part II. For 2, 3, or more different patients one table represents a TCR cluster with closely related TCRs. The columns 1-13 and NN are described below. 1: An arbitrary patient ID. 2-3: Amino acid sequences of both CDR3 chains. 4: The ratio between TCR-clonotype frequencies in tumour versus adjacent non-tumour tissue. 5: The frequency of respective TCR in tumour. 6-9: V/J segments of both chains. 10: HLA type(s) (I/II) in 4-digit resolution. 11-13: Respective T-cell activation marker frequencies. They are measured with single-cell sequencing gene expression technology or, if applicable, with cell sorting technology and respective clonotype frequencies are derived from TCR sequencing data. NN: Any marker for T-cell activation might be used the same way.

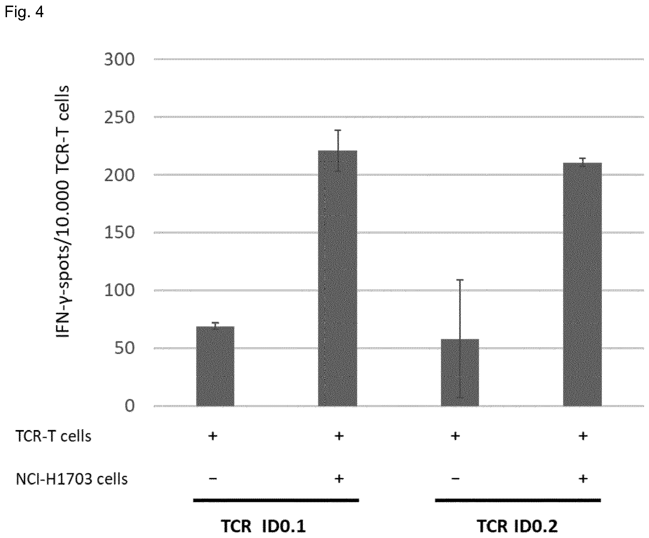

Fig. 4: shows CD8+ T cells from healthy donors were depleted from endogenous TCRs by CRISPR/CAS9-mediated knockout and transduced with TCRs from Cluster 0 clonotypes. Transduced TCR-T cells were then tested against seven NSCLC cell lines by IFN-γ-Elispot assay (not shown). Of the three NSCLC lines expressing the cluster 0-relevant MHC-allele HLA-B*08:01, only NCI-H1703 was recognized by TCR-T cells. In addition to NCI-H1703 cells, TCR-T cells also recognized K562 cells transduced with HLA-B*08:01 (but not wildtype K562 cells which are HLA free) and HLA-B*08:01-expressing lymphoblastoid cell lines (LCLs) (not shown). Reactivity against NSCLC cells could be blocked by pan-HLA-specific antibody indicating peptide-MHC-restricted reactivity (not shown).

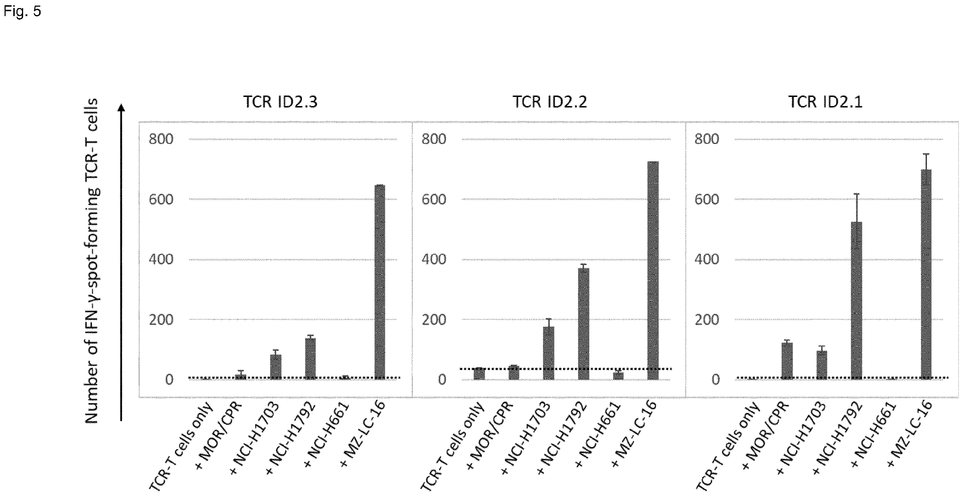

Fig. 5: shows CD8+ T cells from healthy donors were depleted from endogenous TCRs by CRISPR/CAS9-mediated knockout and transduced with TCRs from Cluster 2 clonotypes. TCR-transduced (TCR-)T cells were tested against five HLA-A*02:01-positive NSCLC cell lines by IFN-γ-Elispot assay. All TCR-T cells recognized MZ-LC-16, NCI-H1703 and NCI-H1792 above background reactivity (TCR-T cells only, dotted lines). TCR-ID2.1-transduced TCR-T cells showed a minor response against MOR/CPR in addition. Further testing for cross-reactivity of TCR-T cells revealed that K562 cells transduced with HLA-A*02:01 (but not wildtype K562 cells) and HLA-A*02:01-positive T2-cells were recognized as well (not shown). Reactivity against NSCLC cells could be blocked by HLA-A02-specific antibody indicating peptide-MHC-restricted reactivity (not shown).

### Examples

### A. 1: Preparation of Non-small cell lung cancer (NSCLC) tumor- and normal lung tissue specimen and TCRSafe analysis

Each tumor specimen is dissected free of surrounding normal tissue and necrotic areas. Approx. 1 g cubes from tumor and normal lung tissue are cut into small chunks measuring about 2-3 mm in each dimension. Sliced tumor (and also non-tumor) biopsies are subjected to a commercial mechanical/ enzymatic tissue dissociation system (GentleMACS, Miltenyi Biotec, Bergisch-Gladbach, Germany), using the Tumor Dissociation Kit (Miltenyi Biotech) and following the manufacturer's instructions. After GentleMACS disaggregation, cell suspensions are passed through 70-µm cell strainers. Aliquots of tumor- and lung cells are taken and cryopreserved in 10% DMSO (Sigma-Aldrich) and 90% FCS (Life Technologies) for later use. The remaining cell suspensions are subjected to density gradient centrifugation using a 40%/80% step gradient of Percoll^{®} (GE Healthcare Europe GmbH) in PBS/RPMI 1640. T-lymphocytes are harvested from interphases and washed in complete medium (RPMI 1640, Lonza). Subsequently, tumor- infiltrating T-lymphocytes (TIL) and lymphocytes from normal lung tissue are placed in 24-well tissue culture plates with 2 mL of recovery medium (RM) at a concentration of 0.5x106 cells/ml. RM is RPMI 1640 supplemented with 25 mM HEPES pH 7.2 and L-glutamine (Lonza), 100 IU/mL penicillin, 100 mg/mL streptomycin, and 50 mM beta-mercaptoethanol (ThermoFisher Scientific, Waltham, Massachusetts, USA), supplemented with 10% autologous human serum. Plates are placed in a humidified 37°C incubator with 5% CO2 and cultured overnight. The next day, cells are harvested and pooled from the TIL- and normal lung cultures and the following subpopulations isolated via FACS:
- CD4+ T cells
- CD8+ T cells
- PD1+ cells (from TIL only)
- PD1- negative cells (from TIL only)

From subpopulations, genomic DNA is extracted and subjected to TCRsafe analysis (as disclosed in WO 2014/096394 A1). The resulting T-cell clonotype frequencies are compared among subpopulations and tumor-specific clonotypes identified as detailed in WO 2017/025564 A1.

All subsequent steps for these examples refer to CD8+ T-cells isolated from tumour and non-tumour tissues as described above.

### A.2: T cell receptor (TCR) α/β pairing using 10x Genomics high throughput single-cell sequencing

Starting from TIL single-cell suspensions, 5000 - 10000 T-cells are subjected to high throughput single-cell RNASeq analysis using the 10x Genomics Chromium Next GEM Single Cell V(D)J Reagent Kit in combination with the Chromium Single Cell V(D)J Enrichment Kit (Human). 10x Genomics^{®} GemCodeTM Technology disperses thousands of individual cells into Gel Bead-in-EMulsion (GEM) droplets. GEM-captured single cells are lysed and upon GEM-solution, barcoded primers attached to the beads, oligos, master mix, and lysed cell components are mixed, and through RT-PCR, full-length oligo-dT-primed cDNA-libraries are generated. First-strand cDNA synthesis by using a template switch mechanism is completed including the barcoded sequence attached to the beads. All cDNA molecules within a single GEM are labelled with the same barcode. GEMs are broken down and further library preparations are continued as bulk reactions. After cDNA-clean up, the Chromium Single Cell V(D)J Enrichment Kit effectively amplifies TCR sequences and generates sequencing libraries compatible with Illumina sequencing. In combination with whole cDNA amplification, Illumina sequencing reveals for each single T cell analyzed the paired α/β TCR sequences and the corresponding whole transcriptome per cell. Both kits, the 10x Genomics Chromium Next GEM Single Cell V(D)J Reagent Kit and the Chromium Single Cell V(D)J Enrichment Kit (Human) are used according to the manufacturer's recommendations.

Nucleotide sequences identical between TCRs described under A.1 above and TCRs from the single-cell VDJ pairing are used to establish the full annotation of TCRs with respect to alpha- and beta chains, frequencies and tumour specificity.

### A.3: Clustering of TCRs (TCRpolyClust)

As described in the schema of TCRpolyClust method once the combination of A.1 and A.2 is established a subsequent TCR cluster analysis identifies patients with common TCRs and matching HLA-types enabling the screening for shared tumor antigens.

### Synthesis, cloning, and ectopic expression of cluster TCRs in T cells isolated from autologous patients or healthy donors

Paired cluster TCRs are codon-optimized, synthesized, and cloned as bicistronic chimeric constructs (βTCR-VDJ-mC_P2A-element_αTCR-VJ-mC; mC represent murine constant domains) into retroviral (or comparable) expression vectors for transduction of autologous or allogeneic T cells from blood of the respective patients or healthy donors. Recipient T cells are pretreated with CRISPR/Cas9 to knock-out endogenous TCRs to prevent off-target immune reactions mediated by mixed TCR-dimers (endogenous x exogenous chains, in autologous and allogeneic settings) or allo-responses by endogenous TCRs (in allogeneic settings). Said chimeric (c)TCR-recombinant T cells are expanded in vitro and applied to functional experiments such as recognition of autologous tumor cells (if available), allogeneic tumor cell lines, and/or antigen-screenings as described below.

### Targeted approaches for the screening of shared tumor antigens

Comparative whole-exome (WES) and whole transcriptome (WTS) sequencing of tumor- and corresponding normal tissue genomic and total-RNA including samples from all patients of a respective TCR cluster are applied to identify shared neoantigens (SNV, MNV, InDels, fusion gene products, structural alterations), aberrantly expressed canonical genes (cancer/germline- and overexpressed antigens), and aberrantly expressed and translated non-canonical transcripts (dark matter transcripts or cryptic transcripts). Candidates of all categories are then tested for recognition by the cTCR-transduced recombinant T cells. Antigen formats are either expression plasmids encoding full-length antigen-cDNAs or tandem minigenes (TMGs) encoding only the peptide-coding regions with immunogenic potential of the candidate antigens. Both formats are tested by co-transfection of antigen- and HLA-cDNA-encoding plasmids in 293T- or COS-7 cells and subjecting the transfectants to recognition testing by the T cells in IFN-y ELISpot assays. Alternatively, antigenic peptide candidates can be predicted for binding to the relevant HLA alleles using public prediction algorithms (IEDB, NetMHC), the peptides synthesized and pulsed onto HLA-matched antigen-presenting cells. The latter are then subjected to ELISpot assays testing their recognition by the recombinant T cells.

### Tumor cDNA-expression-library screening approaches

The targeted identification of antigen candidates may not be equally effective for all antigen categories. For example, while the screening for non-synonymous somatic mutations of tumor cells using whole-exome- and -transcriptome sequencing is sensitive, highly reproducible, and produces a quantitative list of potential neoantigens, the identification of cryptic translatable transcripts (dark matter antigens) is less efficient due to a general lack of specific traits to identify them reliably. This dilemma can be solved by probing the complete transcriptome of tumor cells by cDNA-expression-library screening approaches. Either from sorted autologous tumor cells or from HLA-matched tumor cell lines, shown before to be recognized by cTCR-transduced T cells, cDNA-expression libraries generated from total-RNA are co-expressed with appropriate HLA-alleles in antigen-presenting cells (293T- or COS-7 cells). Transfectants are then tested for recognition by cTCR-transduced T cells via ELISpot assays. To have a chance to get expression even of rare transcripts after transfection, the screening procedure requires a high throughput approach testing a highly fractionated cDNA-library. For this purpose, a cDNA-library is produced consisting e.g. of 2000 pools of 100 cDNAs per well prepared in a 96-well plate format. Transfections and ELISpot assays using the cTCR-transduced T cells as effector cells are conducted in this 96-well format and from recognized pools of 100, step-wise reduction of pools (e.g. 10 cDNAs/pool and well, cDNA-clones/pool and well) and testing will result in the selection of antigen-encoding cDNA-clones.

Because a sufficiently pure and large enough population of viable autologous tumor cells for RNA-isolation and cDNA-library preparation can be obtained only for a minority of patients, a pre-screening for recognition of type-matched tumor cell lines can be conducted. The cell lines can either be selected for shared expression of HLA alleles or be transduced with HLAs of interest. Recognized cell lines are used as proof for the existence of the common antigen and as sources for RNA-extraction and cDNA-library generation.

### Sequences

TCR sequences are constructed as follows (from N to C terminus):
Block-V_CDR3_Block-J/C

| | |
|---|---|
| Block-V | Constant part of TCR-chain (alpha or beta) from start codon (M) to the variable CDR3 region |
| CDR3 | Complementarity-determining region 3 |
| Block-J/C | The constant region from CDR3 through J-segment to the respective C-element, ending with a stop (*) codon |

**Table 1: Cluster ID 0**

| SEQ-ID | CDR3 **beta:** Amino acid seq. | CDR3 **alpha:** Amino acid seq. | HLA_B_I | HLA_C_II |
|---|---|---|---|---|
| 0.1 | CASSPGPNYEQYF (SEQ ID NO: 38) | CAGAYNQGGKLIF (SEQ ID NO: 6) | B*8:01 | C*07:01 |
| 0.2 | CASSAGPNYEQYF (SEQ ID NO: 41) | CAASFNQGGKLIF (SEQ ID NO: 1) | B*08:01 | C*07:01 |
| 0.3 | CASSLGPNYEQYV (SEQ ID NO: 20) | CAASSNQGGKLIF (SEQ ID NO: 59) | B*08:01 | C*07:01 |

### Cluster ID 0:

Cluster 0 is associated with **HLA-B^{∗}08:01 and HLA-C^{∗}07:01**
Beta chain, TRB: **TRBV7-6^{∗}01, TRBJ2-7^{∗}01, TRBC2^{∗}02**
**Cluster 0 Block V** 
ATGGGCACCAGTCTCCTATGCTGGGTGGTCCTGGGTTTCCTAGGGACAGATCACACAGGTGCTGGAGTCTCCC
AGTCTCCCAGGTACAAAGTCACAAAGAGGGGACAGGATGTAGCTCTCAGGTGTGATCCAATTTCGGGTCATGT
ATCCCTTTATTGGTACCGACAGGCCCTGGGGCAGGGCCCAGAGTTTCTGACTTACTTCAATTATGAAGCCCAAC
AAGACAAATCAGGGCTGCCCAATGATCGGTTCTCTGCAGAGAGGCCTGAGGGATCCATCTCCACTCTGACGAT
CCAGCGCACAGAGCAGCGGGACTCGGCCATGTATCGC (SEQ ID NO: 96)
 **Seq-IDO.1b**
TGTGCCAGCAGCCCCGGACCCAACTACGAGCAGTACTTC (SEQ ID NO: 74)
**Seq-IDO.2b**
TGTGCCAGCAG**TG**CAGG**G**CCCAA**T**TACGAGCAGTACTTC (SEQ ID NO: 139)
**Seq-ID0.3b**
TGTGCCAGCAGC**TTA**GG**C**CC**G**AA**T**TACGAGCAGTAC**G**TC (SEQ ID NO: 125)
**Cluster 0 Block J/C** 
GGGCCGGGCACCAGGCTCACGGTCACAGAGGACCTGAAAAACGTGTTCCCACCCGAGGTCGCTGTGTTTGAG
CCATCAGAAGCAGAGATCTCCCACACCCAAAAGGCCACACTGGTGTGCCTGGCCACAGGCTTCTACCCCGACC
ACGTGGAGCTGAGCTGGTGGGTGAATGGGAAGGAGGTGCACAGTGGGGTCAGCACAGACCCGCAGCCCCTC
AAGGAGCAGCCCGCCCTCAATGACTCCAGATACTGCCTGAGCAGCCGCCTGAGGGTCTCGGCCACCTTCTGGC
AGAACCCCCGCAACCACTTCCGCTGTCAAGTCCAGTTCTACGGGCTCTCGGAGAATGACGAGTGGACCCAGGA
TAGGGCCAAACCTGTCACCCAGATCGTCAGCGCCGAGGCCTGGGGTAGAGCAGACTGTGGCTTCACCTCCGA
GTCTTACCAGCAAGGGGTCCTGTCTGCCACCATCCTCTATGAGATCTTGCTAGGGAAGGCCACCTTGTATGCCG
TGCTGGTCAGTGCCCTCGTGCTGATGGCCATGGTCAAGAGAAAGGATTCCAGAGGCTAG (SEQ ID NO:
151)
 **Cluster 0 Block V** 
MGTSLLCWVVLGFLGTDHTGAGVSQSPRYKVTKRGQDVALRCDPISGHVSLYWYRQALGQGPEFLTYFNYEAQQ
DKSGLPNDRFSAERPEGSISTLTIQRTEQRDSAMYR (SEQ ID NO: 23)
 **Seq-IDO.1b**
CASSPGPNYEQYF (SEQ ID NO: 38)
**Seq-IDO.2b**
CASSAGPNYEQYF (SEQ ID NO: 41)
**Seq-IDO.3b**
CASSLGPNYEQYV (SEQ ID NO: 20)
**Cluster 0 Block J/C** 
GPGTRLTVTEDLKNVFPPEVAVFEPSEAEISHTQKATLVCLATGFYPDHVELSWWVNGKEVHSGVSTDPQPLKEQP
ALNDSRYCLSSRLRVSATFWQNPRNHFRCQVQFYGLSENDEWTQDRAKPVTQIVSAEAWGRADCGFTSESYQQG
VLSATILYEILLGKATLYAVLVSALVLMAMVKRKDSRG (SEQ ID NO: 33)
 Alpha-chain, TRA: **TRAV13-1^{∗}01, TRAJ23^{∗}01, TRAC^{∗}01**
**Cluster 0 Block V** 
ATGACATCCATTCGAGCTGTATTTATATTCCTGTGGCTGCAGCTGGACTTGGTGAATGGAGAGAATGTGGAGC
AGCATCCTTCAACCCTGAGTGTCCAGGAGGGAGACAGCGCTGTTATCAAGTGTACTTATTCAGACAGTGCCTC
AAACTACTTCCCTTGGTATAAGCAAGAACTTGGAAAAAGACCTCAGCTTATTATAGACATTCGTTCAAATGTGG
GCGAAAAGAAAGACCAACGAATTGCTGTTACATTGAACAAGACAGCCAAACATTTCTCCCTGCACATCACAGA
GACCCAACCTGAAGACTCGGCTGTCTACTTC (SEQ ID NO: 108)
 **Seq-IDO.1a**
TGTGCAGGGGCGTATAACCAGGGAGGAAAGCTTATCTTC (SEQ ID NO: 85)
**Seq-ID0.2a**
TGTGCAG**CAAGT**T**T**TAACCAGGGAGGAAAGCTTATCTTC (SEQ ID NO: 106)
**Seq-ID0.3a**
TGTGCAG**C**A**AGTAG**TAACCAGGGAGGAAAGCTTATCTTC (SEQ ID NO: 123)
**Cluster 0 Block J/C** 
GGACAGGGAACGGAGTTATCTGTGAAACCCAATATCCAGAACCCTGACCCTGCCGTGTACCAGCTGAGAGACT
CTAAATCCAGTGACAAGTCTGTCTGCCTATTCACCGATTTTGATTCTCAAACAAATGTGTCACAAAGTAAGGATT
CTGATGTGTATATCACAGACAAAACTGTGCTAGACATGAGGTCTATGGACTTCAAGAGCAACAGTGCTGTGGC
CTGGAGCAACAAATCTGACTTTGCATGTGCAAACGCCTTCAACAACAGCATTATTCCAGAAGACACCTTCTTCC
CCAGCCCAGAAAGTTCCTGTGATGTCAAGCTGGTCGAGAAAAGCTTTGAAACAGATACGAACCTAAACTTTCA
AAACCTGTCAGTGATTGGGTTCCGAATCCTCCTCCTGAAAGTGGCCGGGTTTAATCTGCTCATGACGCTGCGGC
TGTGGTCCAGCTGA (SEQ ID NO: 87)
 **Cluster 0 Block V** 

 (SEQ ID NO: 67)
 **Seq-IDO.1a**
CAGAYNQGGKLIF (SEQ ID NO: 6)
**Seq-ID0.2a**
CAASFNQGGKLIF (SEQ ID NO: 1)
**Seq-IDO.3a**
CAASSNQGGKLIF (SEQ ID NO: 59)
**Cluster 0 Block J/C**

GQGTELSVKPNIQNPDPAVYQLRDSKSSDKSVCLFTDFDSQTNVSQSKDSDVYITDKTVLDMRSMDFKSNSAVAW
SNKSDFACANAFNNSIIPEDTFFPSPESSCDVKLVEKSFETDTNLNFQNLSVIGFRILLLKVAGFNLLMTLRLWSS
(SEQ ID NO: 4)

**Table2: Cluster ID 1:**

| SEQ-ID | CDR3 **beta:** Amino acid seq. | CDR3 **alpha:** Amino acid seq. | HLA_A_I | HLA_A_II |
|---|---|---|---|---|
| 1.1 | CASSFVSGTDTQYF | CAFMARGSTLGRLYF | A*02:01 | A*02:01 |
| | (SEQ ID NO: 47) | (SEQ ID NO: 58) | | |
| 1.2 | CASSFVSGTDTQYF (SEQ ID NO: 47) | CAFMERGSTLGRLYF (SEQ ID NO: 12) | A*02:01 | |
| 1.3 | CASSFLSGTDTQYF (SEQ ID NO: 73) | CAFLTRGSTLGRLYF (SEQ ID NO: 18) | | |
| 1.4 | CASSFLSGTDTQYF (SEQ ID NO: 73) | CAFMPRGSTLGRLYF (SEQ ID NO: 55) | | A*68:02 |
| 1.5 | CASSFLAGTDTQYF (SEQ ID NO: 72) | CAPLPRGSTLGRLYF (SEQ ID NO: 26) | A*02:01 | |
| 1.6 | CASSFVSGTDTQYF (SEQ ID NO: 47) | CALLSRGSTLGRLYF (SEQ ID NO: 11) | | A*02:01 |

### Cluster ID 1:

Cluster 1 is associated with the **HLA-A^{∗}02:01-supertype (HLA-A^{∗}02:01/68:02)**
Beta chain, TRB: **TRBV28^{∗}01, TRBJ2-3^{∗}01, TRBC2^{∗}01**
**Cluster 1 Block V** 
ATGGGAATCAGGCTCCTCTGTCGTGTGGCCTTTTGTTTCCTGGCTGTAGGCCTCGTAGATGTGAAAGTAACCCA
GAGCTCGAGATATCTAGTCAAAAGGACGGGAGAGAAAGTTTTTCTGGAATGTGTCCAGGATATGGACCATGA
AAATATGTTCTGGTATCGACAAGACCCAGGTCTGGGGCTACGGCTGATCTATTTCTCATATGATGTTAAAATGA
AAGAAAAAGGAGATATTCCTGAGGGGTACAGTGTCTCTAGAGAGAAGAAGGAGCGCTTCTCCCTGATTCTGG
AGTCCGCCAGCACCAACCAGACATCTATGTACCTC (SEQ ID NO: 97)
 **Seq-ID1.1b**
TGTGCCAGCAGTTTCGTCAGCGGCACAGATACGCAGTATTTT (SEQ ID NO: 115)
**Seq-ID1.2b**
TGTGCCAGCAGTTT**T**GT**G**AGCGGCACAGATACGCAGTATTTT (SEQ ID NO: 88)
**Seq-ID1.3b**
TGTGCCAGCAGTTT**TC**T**T**AGCGGCACAGATACGCAGTATTTT (SEQ ID NO: 157)
**Seq-ID1.4b**
TGTGCCAGCAGTTT**TC**TT**TCAG**GCACAGATACGCAGTATTTT (SEQ ID NO: 101)
**Seq-ID1.5b**
TGTGCCAGCAGTTTC**C**T**AGCG**GGCACAGATACGCAGTATTTT (SEQ ID NO: 136)
**Seq-ID1.6b**
TGTGCCAGCAGTTTCGT**TTCA**GGCACAGATACGCAGTATTTT (SEQ ID NO: 133)
**Cluster 1 Block J/C** 
GGCCCAGGCACCCGGCTGACAGTGCTCGAGGACCTGAAAAACGTGTTCCCACCCAAGGTCGCTGTGTTTGAGC
CATCAGAAGCAGAGATCTCCCACACCCAAAAGGCCACACTGGTGTGCCTGGCCACAGGCTTCTACCCCGACCA
CGTGGAGCTGAGCTGGTGGGTGAATGGGAAGGAGGTGCACAGTGGGGTCAGCACAGACCCGCAGCCCCTCA
AGGAGCAGCCCGCCCTCAATGACTCCAGATACTGCCTGAGCAGCCGCCTGAGGGTCTCGGCCACCTTCTGGCA
GAACCCCCGCAACCACTTCCGCTGTCAAGTCCAGTTCTACGGGCTCTCGGAGAATGACGAGTGGACCCAGGAT
AGGGCCAAACCTGTCACCCAGATCGTCAGCGCCGAGGCCTGGGGTAGAGCAGACTGTGGCTTCACCTCCGAG
TCTTACCAGCAAGGGGTCCTGTCTGCCACCATCCTCTATGAGATCTTGCTAGGGAAGGCCACCTTGTATGCCGT
GCTGGTCAGTGCCCTCGTGCTGATGGCCATGGTCAAGAGAAAGGATTCCAGAGGCTAG (SEQ ID NO: 86)
 **Cluster 1 Block V**
MGIRLLCRVAFCFLAVGLVDVKVTQSSRYLVKRTGEKVFLECVQDMDHENMFWYRQDPGLGLRLIYFSYDVKMKE KGDIPEGYSVSREKKERFSLILESASTNQTSMYL (SEQ ID NO: 64)
**Seq-ID1.1b**
CASSFVSGTDTQYF (SEQ ID NO: 47)
**Seq-ID1.2b**
CASSFVSGTDTQYF (SEQ ID NO: 47)
**Seq-ID1.3b**
CASSFLSGTDTQYF (SEQ ID NO: 73)
**Seq-ID1.4b**
CASSF**L**SGTDTQYF (SEQ ID NO: 73)
**Seq-ID1.5b**
CASSF**LA**GTDTQYF (SEQ ID NO: 72)
**Seq-ID1.6b**
CASSFVSGTDTQYF (SEQ ID NO: 47)
**Cluster 1 J/C** 
GPGTRLTVLEDLKNVFPPKVAVFEPSEAEISHTQKATLVCLATGFYPDHVELSWWVNGKEVHSGVSTDPQPLKEQP
ALNDSRYCLSSRLRVSATFWQNPRNHFRCQVQFYGLSENDEWTQDRAKPVTQIVSAEAWGRADCGFTSESYQQG
VLSATILYEILLGKATLYAVLVSALVLMAMVKRKDSRG (SEQ ID NO: 56)
 Alpha-chain, TRA: **TRAV24^{∗}01, TRAJ18^{∗}01, TRAC^{∗}01**
**Cluster 1 Block V** 
ATGGAGAAGAATCCTTTGGCAGCCCCATTACTAATCCTCTGGTTTCATCTTGACTGCGTGAGCAGCATACTGAA
CGTGGAACAAAGTCCTCAGTCACTGCATGTTCAGGAGGGAGACAGCACCAATTTCACCTGCAGCTTCCCTTCCA
GCAATTTTTATGCCTTACACTGGTACAGATGGGAAACTGCAAAAAGCCCCGAGGCCTTGTTTGTAATGACTTTA
AATGGGGATGAAAAGAAGAAAGGACGAATAAGTGCCACTCTTAATACCAAGGAGGGTTACAGCTATTTGTAC
ATCAAAGGATCCCAGCCTGAAGACTCAGCCACATACCTC (SEQ ID NO: 131)
 **Seq-ID1.1a**
TGTGCCTTTATGGCCAGAGGCTCAACCCTGGGGAGGCTATACTTT (SEQ ID NO: 134)
**Seq-ID1.2a**
TGTGCCTTTATGG**AG**AGAGGCTCAACCCTGGGGAGGCTATACTTT (SEQ ID NO: 124)
**Seq-ID1.3a**
TGTGCCTTT**C**T**TA**CCAGAGGCTCAACCCTGGGGAGGCTATACTTT (SEQ ID NO: 119)
**Seq-ID1.4a**
TGTGCCTTTATG**C**CCAGAGGCTCAACCCTGGGGAGGCTATACTTT (SEQ ID NO: 147)
**Seq-ID1.5a**
TGTGCC**CCCC**T**CC**CGAGAGGCTCAACCCTGGGGAGGCTATACTTT (SEQ ID NO: 102)
**Seq-ID1.6a**
TGTGCCTT**AT**T**AAG**CAGAGGCTCAACCCTGGGGAGGCTATACTTT (SEQ ID NO: 132)
**Cluster 1 Block J/C** 
GGAAGAGGAACTCAGTTGACTGTCTGGCCTGATATCCAGAACCCTGACCCTGCCGTGTACCAGCTGAGAGACT
CTAAATCCAGTGACAAGTCTGTCTGCCTATTCACCGATTTTGATTCTCAAACAAATGTGTCACAAAGTAAGGATT
CTGATGTGTATATCACAGACAAAACTGTGCTAGACATGAGGTCTATGGACTTCAAGAGCAACAGTGCTGTGGC
CTGGAGCAACAAATCTGACTTTGCATGTGCAAACGCCTTCAACAACAGCATTATTCCAGAAGACACCTTCTTCC
CCAGCCCAGAAAGTTCCTGTGATGTCAAGCTGGTCGAGAAAAGCTTTGAAACAGATACGAACCTAAACTTTCA
AAACCTGTCAGTGATTGGGTTCCGAATCCTCCTCCTGAAAGTGGCCGGGTTTAATCTGCTCATGACGCTGCGGC
TGTGGTCCAGCTGA (SEQ ID NO: 138)
 **Cluster 1 Block V** 
MEKNPLAAPLLILWFHLDCVSSILNVEQSPQSLHVQEGDSTNFTCSFPSSNFYALHWYRWETAKSPEALFVMTLNG
DEKKKGRISATLNTKEGYSYLYIKGSQPEDSATYL (SEQ ID NO: 3)
 **Seq-ID1.1a**
CAFMARGSTLGRLYF (SEQ ID NO: 58)
**Seq-ID1.2a**
CAFM**E**RGSTLGRLYF (SEQ ID NO: 12)
**Seq-ID1.3a**
CAF**LT**RGSTLGRLYF (SEQ ID NO: 18)
**Seq-ID1.4a**
CAFM**P**RGSTLGRLYF (SEQ ID NO: 55)
**Seq-ID1.5a**
CA**PLP**RGSTLGRLYF (SEQ ID NO: 26)
**Seq-ID1.6a**
CA**LLS**RGSTLGRLYF (SEQ ID NO: 11)
**Cluster 1 Block J/C**

GRGTQLTVWPDIQNPDPAVYQLRDSKSSDKSVCLFTDFDSQTNVSQSKDSDVYITDKTVLDMRSMDFKSNSAVA
WSNKSDFACANAFNNSIIPEDTFFPSPESSCDVKLVEKSFETDTNLNFQNLSVIGFRILLLKVAGFNLLMTLRLWSS
(SEQ ID NO: 53)

**Table 3: Cluster ID 31:**

| SEQ-ID | CDR3 **beta:** Amino acid seq. | CDR3 **alpha:** Amino acid seq. | HLA_A_I | HLA_C_II |
|---|---|---|---|---|
| 31.1 | CASSLDGMNTEAFF (SEQ ID NO: 28) | CAARLTGTASKLTF (SEQ ID NO: 63) | A*02:05 | C*07:01 |
| 31.2 | CASSLDGMNTEAFF (SEQ ID NO: 28) | CAARNAGTASKLTF (SEQ ID NO: 71) | A*02:35 | C*07:04 |
| 31.3 | CASSLDGMNTEAFF | CAARNTGTASKLTF | A*02:01 | C*07:01 |
| | (SEQ ID NO: 28) | (SEQ ID NO: 62) | | |

### Cluster ID 31:

Cluster 31 is associated with the **HLA-A**^{∗}**02:01-supertype (HLA-A**^{∗}**02:01/02:35/02:05) or HLA-C**^{∗}**07:01/07:04**
Beta chain, TRB: **TRBV5-1**^{∗}**01, TRBJ1-1**^{∗}**01, TRBC1**^{∗}**01**
**Cluster 31 Block V** 
ATGGGCTCCAGGCTGCTCTGTTGGGTGCTGCTTTGTCTCCTGGGAGCAGGCCCAGTAAAGGCTGGAGTCACTC
AAACTCCAAGATATCTGATCAAAACGAGAGGACAGCAAGTGACACTGAGCTGCTCCCCTATCTCTGGGCATAG
GAGTGTATCCTGGTACCAACAGACCCCAGGACAGGGCCTTCAGTTCCTCTTTGAATACTTCAGTGAGACACAG
AGAAACAAAGGAAACTTCCCTGGTCGATTCTCAGGGCGCCAGTTCTCTAACTCTCGCTCTGAGATGAATGTGA
GCACCTTGGAGCTGGGGGACTCGGCCCTTTATCTT (SEQ ID NO: 116)
 **Seq-ID31.1b**
TGCGCCAGCAGTTTGGACGGGATGAACACTGAAGCTTTCTTT (SEQ ID NO: 98)
**Seq-ID31.2b**
TGCGCCAGCAG**C**TTGGACGG**A**ATGAACACTGAAGCTTTCTTT (SEQ ID NO: 83)
**Seq-ID31.3b**
TGCGCCAGCAG**C**TTGGACGG**C**ATGAACACTGAAGCTTTCTTT (SEQ ID NO: 129)
**Cluster 31 Block J/C** 
GGACAAGGCACCAGACTCACAGTTGTAGAGGACCTGAACAAGGTGTTCCCACCCGAGGTCGCTGTGTTTGAG
CCATCAGAAGCAGAGATCTCCCACACCCAAAAGGCCACACTGGTGTGCCTGGCCACAGGCTTCTTCCCTGACC
ACGTGGAGCTGAGCTGGTGGGTGAATGGGAAGGAGGTGCACAGTGGGGTCAGCACGGACCCGCAGCCCCTC
AAGGAGCAGCCCGCCCTCAATGACTCCAGATACTGCCTGAGCAGCCGCCTGAGGGTCTCGGCCACCTTCTGGC
AGAACCCCCGCAACCACTTCCGCTGTCAAGTCCAGTTCTACGGGCTCTCGGAGAATGACGAGTGGACCCAGGA
TAGGGCCAAACCCGTCACCCAGATCGTCAGCGCCGAGGCCTGGGGTAGAGCAGACTGTGGCTTTACCTCGGT
GTCCTACCAGCAAGGGGTCCTGTCTGCCACCATCCTCTATGAGATCCTGCTAGGGAAGGCCACCCTGTATGCTG
TGCTGGTCAGCGCCCTTGTGTTGATGGCCATGGTCAAGAGAAAGGATTTCTGA (SEQ ID NO: 103)
 **Cluster 31 Block V** 
MGSRLLCWVLLCLLGAGPVKAGVTQTPRYLIKTRGQQVTLSCSPISGHRSVSWYQQTPGQGLQFLFEYFSETQRNK
GNFPGRFSGRQFSNSRSEMNVSTLELGDSALYL (SEQ ID NO: 43)
 **Seq-ID31.1b**
CASSLDGMNTEAFF (SEQ ID NO: 28)
**Seq-ID31.2b**
CASSLDGMNTEAFF (SEQ ID NO: 28)
**Seq-ID31.3b**
CASSLDGMNTEAFF (SEQ ID NO: 28)
**Cluster 31 Block J/C** 
GQGTRLTVVEDLNKVFPPEVAVFEPSEAEISHTQKATLVCLATGFFPDHVELSWWVNGKEVHSGVSTDPQPLKEQP
ALNDSRYCLSSRLRVSATFWQNPRNHFRCQVQFYGLSENDEWTQDRAKPVTQIVSAEAWGRADCGFTSVSYQQG
VLSATILYEILLGKATLYAVLVSALVLMAMVKRKDF (SEQ ID NO: 37)
 Alpha-chain, TRA: **TRAV29/DV^{∗}01, TRAJ44^{∗}01, TRAC^{∗}01**
**Cluster 31 Block V** 
ATGGCCATGCTCCTGGGGGCATCAGTGCTGATTCTGTGGCTTCAGCCAGACTGGGTAAACAGTCAACAGAAGA
ATGATGACCAGCAAGTTAAGCAAAATTCACCATCCCTGAGCGTCCAGGAAGGAAGAATTTCTATTCTGAACTG
TGACTATACTAACAGCATGTTTGATTATTTCCTATGGTACAAAAAATACCCTGCTGAAGGTCCTACATTCCTGAT
ATCTATAAGTTCCATTAAGGATAAAAATGAAGATGGAAGATTCACTGTCTTCTTAAACAAAAGTGCCAAGCACC
TCTCTCTGCACATTGTGCCCTCCCAGCCTGGAGACTCTGCAGTGTACTTC (SEQ ID NO: 79)
 **Seq-ID31.1a**
TGTGCAGCAAGACTTACCGGCACTGCCAGTAAACTCACCTTT (SEQ ID NO: 143)
**Seq-ID31.2a**
TGTGCAGCAAG**GAA**T**G**CCGGCACTGCCAGTAAACTCACCTTT (SEQ ID NO: 148)
**Seq-ID31.3a**
TGTGCAGCAAG**GAA**TACCGGCACTGCCAGTAAACTCACCTTT (SEQ ID NO: 152)
**Cluster 31 Block J/C** 
GGGACTGGAACAAGACTTCAGGTCACGCTCGATATCCAGAACCCTGACCCTGCCGTGTACCAGCTGAGAGACT
CTAAATCCAGTGACAAGTCTGTCTGCCTATTCACCGATTTTGATTCTCAAACAAATGTGTCACAAAGTAAGGATT
CTGATGTGTATATCACAGACAAAACTGTGCTAGACATGAGGTCTATGGACTTCAAGAGCAACAGTGCTGTGGC
CTGGAGCAACAAATCTGACTTTGCATGTGCAAACGCCTTCAACAACAGCATTATTCCAGAAGACACCTTCTTCC
CCAGCCCAGAAAGTTCCTGTGATGTCAAGCTGGTCGAGAAAAGCTTTGAAACAGATACGAACCTAAACTTTCA
AAACCTGTCAGTGATTGGGTTCCGAATCCTCCTCCTGAAAGTGGCCGGGTTTAATCTGCTCATGACGCTGCGGC
TGTGGTCCAGCTGA (SEQ ID NO: 158)
 **Cluster 31 Block V** 
MAMLLGASVLILWLQPDWVNSQQKNDDQQVKQNSPSLSVQEGRISILNCDYTNSMFDYFLWYKKYPAEGPTFLIS
ISSIKDKNEDGRFTVFLNKSAKHLSLHIVPSQPGDSAVYF (SEQ ID NO: 54)
 **Seq-ID31.1a**
CAARLTGTASKLTF (SEQ ID NO: 63)
**Seq-ID31.2a**
CAAR**NA**GTASKLTF (SEQ ID NO: 71)
**Seq-ID31.3a**
CAAR**N**TGTASKLTF (SEQ ID NO: 62)
**Cluster 31 Block J/C**

GTGTRLQVTLDIQNPDPAVYQLRDSKSSDKSVCLFTDFDSQTNVSQSKDSDVYITDKTVLDMRSMDFKSNSAVAW
SNKSDFACANAFNNSIIPEDTFFPSPESSCDVKLVEKSFETDTNLNFQNLSVIGFRILLLKVAGFNLLMTLRLWSS
(SEQ ID NO: 48)

**Table 4: Cluster ID 2:**

| SEQ-ID | CDR3 **beta:** Amino acid seq. | CDR3 **alpha:** Amino acid seq. | HLA_A_I | HLA_A_II |
|---|---|---|---|---|
| 2.1 | CASSEDGMNTEAFF (SEQ ID NO: 19) | CAVLMDSNYQLIW (SEQ ID NO: 51) | A*01:01 | A*02:01 |
| 2.2 | CASSSDGMNTEAFF (SEQ ID NO: 60) | CAVLMDSNYQLIW (SEQ ID NO: 51) | A*01:01 | A*02:01 |
| 2.3 | CASSPDGMNTEAFF (SEQ ID NO: 45) | CALLMDSNYQLIW (SEQ ID NO: 8) | A*01:01 | A*02:01 |

### Cluster ID 2:

Cluster 2 is associated with **HLA-A^{∗}01:01 or HLA-A^{∗}02:01**
Beta chain, TRB: **TRBV10-2, TRBJ1-1, TRBC1^{∗}01**
**Cluster 2 Block V** 
ATGGGCACCAGGCTCTTCTTCTATGTGGCCCTTTGTCTGCTGTGGGCAGGACACAGGGATGCTGGAATCACCC
AGAGCCCAAGATACAAGATCACAGAGACAGGAAGGCAGGTGACCTTGATGTGTCACCAGACTTGGAGCCACA
 
GCTATATGTTCTGGTATCGACAAGACCTGGGACATGGGCTGAGGCTGATCTATTACTCAGCAGCTGCTGATATT
ACAGATAAAGGAGAAGTCCCCGATGGCTATGTTGTCTCCAGATCCAAGACAGAGAATTTCCCCCTCACTCTGG
AGTCAGCTACCCGCTCCCAGACATCTGTGTATTTC (SEQ ID NO: 78)
 **Seq-ID2.1b**
TGCGCCAGCAGTGAGGACGGCATGAACACTGAAGCTTTCTTT (SEQ ID NO: 77)
**Seq-ID2.2b**
TGCGCCAGCAGT**TCC**GACGG**G**ATGAACACTGAAGCTTTCTTT (SEQ ID NO: 107)
**Seq-ID2.3b**
TGCGCCAGCAG**CCCG**GACGGAATG**A**ACACTGAAGCTTTCTTT (SEQ ID NO: 92)
**Cluster 2 Block J/C** 
GGACAAGGCACCAGACTCACAGTTGTAGAGGACCTGAACAAGGTGTTCCCACCCGAGGTCGCTGTGTTTGAG
CCATCAGAAGCAGAGATCTCCCACACCCAAAAGGCCACACTGGTGTGCCTGGCCACAGGCTTCTTCCCTGACC
ACGTGGAGCTGAGCTGGTGGGTGAATGGGAAGGAGGTGCACAGTGGGGTCAGCACGGACCCGCAGCCCCTC
AAGGAGCAGCCCGCCCTCAATGACTCCAGATACTGCCTGAGCAGCCGCCTGAGGGTCTCGGCCACCTTCTGGC
AGAACCCCCGCAACCACTTCCGCTGTCAAGTCCAGTTCTACGGGCTCTCGGAGAATGACGAGTGGACCCAGGA
TAGGGCCAAACCCGTCACCCAGATCGTCAGCGCCGAGGCCTGGGGTAGAGCAGACTGTGGCTTTACCTCGGT
GTCCTACCAGCAAGGGGTCCTGTCTGCCACCATCCTCTATGAGATCCTGCTAGGGAAGGCCACCCTGTATGCTG
TGCTGGTCAGCGCCCTTGTGTTGATGGCCATGGTCAAGAGAAAGGATTTCTGA (SEQ ID NO: 103)
 **Cluster 2 Block V**
MGTRLFFYVALCLLWAGHRDAGITQSPRYKITETGRQVTLMCHQTWSHSYMFWYRQDLGHGLRLIYYSAAADITD KGEVPDGYVVSRSKTENFPLTLESATRSQTSVYF (SEQ ID NO: 30)
**Seq-ID2.1b**
CASSEDGMNTEAFF (SEQ ID NO: 19)
**Seq-ID2.2b**
CASS**S**DGMNTEAFF (SEQ ID NO: 60)
**Seq-ID2.3b**
CASS**P**DGMNTEAFF (SEQ ID NO: 45)
**Cluster 2 Block J/C** 
GQGTRLTVVEDLNKVFPPEVAVFEPSEAEISHTQKATLVCLATGFFPDHVELSWWVNGKEVHSGVSTDPQPLKEQP
ALNDSRYCLSSRLRVSATFWQNPRNHFRCQVQFYGLSENDEWTQDRAKPVTQIVSAEAWGRADCGFTSVSYQQG
VLSATILYEILLGKATLYAVLVSALVLMAMVKRKDF (SEQ ID NO: 37)
 Alpha-chain, TRA: **TRAV21^{∗}01, TRAJ33^{∗}01, TRAC^{∗}01**
**Cluster 2 Block V** 
ATGGAGACCCTCTTGGGCCTGCTTATCCTTTGGCTGCAGCTGCAATGGGTGAGCAGCAAACAGGAGGTGACAC
AGATTCCTGCAGCTCTGAGTGTCCCAGAAGGAGAAAACTTGGTTCTCAACTGCAGTTTCACTGATAGCGCTATT
TACAACCTCCAGTGGTTTAGGCAGGACCCTGGGAAAGGTCTCACATCTCTGTTGCTTATTCAGTCAAGTCAGAG
AGAGCAAACAAGTGGAAGACTTAATGCCTCGCTGGATAAATCATCAGGACGTAGTACTTTATACATTGCAGCT
TCTCAGCCTGGTGACTCAGCCACCTACCTC (SEQ ID NO: 145)
 **Seq-ID2.1a**
TGTGCTGTCCTAATGGATAGCAACTATCAGTTAATCTGG (SEQ ID NO: 120)
**Seq-ID2.2a**
TGTGCTGTC**T**TAATGGATAGCAACTATCAGTTAATCTGG (SEQ ID NO: 146)
**Seq-ID2.3a**
TGTGCT**T**T**A**CT**C**ATGGATAGCAACTATCAGTTAATCTGG (SEQ ID NO: 90)
**Cluster 2 Block J/C** 
GGCGCTGGGACCAAGCTAATTATAAAGCCAGATATCCAGAACCCTGACCCTGCCGTGTACCAGCTGAGAGACT
CTAAATCCAGTGACAAGTCTGTCTGCCTATTCACCGATTTTGATTCTCAAACAAATGTGTCACAAAGTAAGGATT
CTGATGTGTATATCACAGACAAAACTGTGCTAGACATGAGGTCTATGGACTTCAAGAGCAACAGTGCTGTGGC
CTGGAGCAACAAATCTGACTTTGCATGTGCAAACGCCTTCAACAACAGCATTATTCCAGAAGACACCTTCTTCC
CCAGCCCAGAAAGTTCCTGTGATGTCAAGCTGGTCGAGAAAAGCTTTGAAACAGATACGAACCTAAACTTTCA
AAACCTGTCAGTGATTGGGTTCCGAATCCTCCTCCTGAAAGTGGCCGGGTTTAATCTGCTCATGACGCTGCGGC
TGTGGTCCAGCTGA (SEQ ID NO: 111)
 **Cluster 2 Block V** 
METLLGLLILWLQLQWVSSKQEVTQIPAALSVPEGENLVLNCSFTDSAIYNLQWFRQDPGKGLTSLLLIQSSQREQTS
GRLNASLDKSSGRSTLYIAASQPGDSATYL (SEQ ID NO: 68)
 **Seq-ID2.1a**
CAVLMDSNYQLIW (SEQ ID NO: 51)
**Seq-ID2.2a**
CAVLMDSNYQLIW (SEQ ID NO: 51)
**Seq-ID2.3a**
CALLMDSNYQLIW (SEQ ID NO: 8)
**Cluster 2 Block J/C**

GAGTKLIIKPDIQNPDPAVYQLRDSKSSDKSVCLFTDFDSQTNVSQSKDSDVYITDKTVLDMRSMDFKSNSAVAWS
NKSDFACANAFNNSIIPEDTFFPSPESSCDVKLVEKSFETDTNLNFQNLSVIGFRILLLKVAGFNLLMTLRLWSS
(SEQ ID NO: 70)

**Table 5: Cluster ID 3:**

| SEQ-ID | CDR3 **beta:** Amino acid seq. | CDR3 **alpha:** Amino acid seq. | HLA_A_I | HLA_A_II |
|---|---|---|---|---|
| 3.1 | CSVGAQGTNEKLFF (SEQ ID NO: 15) | CAESTSRGKLIF (SEQ ID NO: 24) | A*02:01 | A*68:01 |
| 3.2 | CSVGSGGTNEKLFF (SEQ ID NO: 27) | CAESTPGGKLIF (SEQ ID NO: 2) | A*02:01 | A*68:01 |
| 3.3 | CSVGTGGTNEKLFF (SEQ ID NO: 31) | CAESSPQGKLIF (SEQ ID NO: 21) | A*01:01 | A*02:01 |
| 3.4 | CSVGTGGTNEKLFF (SEQ ID NO: 31) | CAESTPRGRLMF (SEQ ID NO: 32) | A*02:01 | A*68:01 |
| 3.5 | CSVGSGGTNEKLFF (SEQ ID NO: 27) | CAESTPGGKLIF (SEQ ID NO: 2) | A*02:01 | A*68:01 |
| 3.6 | CSVGTGGTNEKLFF (SEQ ID NO: 31) | CAESSPQGKLIF (SEQ ID NO: 21) | A*01:01 | A*02:01 |
| 3.7 | CSVGTGGTNEKLFF (SEQ ID NO: 31) | CAESTPRGRLMF (SEQ ID NO: 32) | A*02:01 | A*68:01 |

### Cluster ID 3:

Cluster 3 is associated with **HLA-A^{∗}02:01 or the HLA-A^{∗}01-supertype (A^{∗}01:01/68:01)**
Beta chain, TRB: **TRBV29-1^{∗}01, TRBJ1-4^{∗}01, TRBC1^{∗}01**
**Cluster 3 Block V** 
ATGCTGAGTCTTCTGCTCCTTCTCCTGGGACTAGGCTCTGTGTTCAGTGCTGTCATCTCTCAAAAGCCAAGCAG
GGATATCTGTCAACGTGGAACCTCCCTGACGATCCAGTGTCAAGTCGATAGCCAAGTCACCATGATGTTCTGGT
ACCGTCAGCAACCTGGACAGAGCCTGACACTGATCGCAACTGCAAATCAGGGCTCTGAGGCCACATATGAGA
GTGGATTTGTCATTGACAAGTTTCCCATCAGCCGCCCAAACCTAACATTCTCAACTCTGACTGTGAGCAACATG
AGCCCTGAAGACAGCAGCATATATCTC (SEQ ID NO: 112)
 **Seq-ID3.1b**
TGCAGCGTTGGGGCTCAGGGAACTAATGAAAAACTGTTTTTT (SEQ ID NO: 84)
**Seq-ID3.2b**
TGCAGCGTTGGG**T**C**CGG**GGG**C**ACTAATGAAAAACTGTTTTTT (SEQ ID NO: 144)
**Seq-ID3.3b**
TGCAGCGT**C**GG**AA**C**AGG**GGG**G**ACTAATGAAAAACTGTTTTTT (SEQ ID NO: 89)
**Seq-ID3.4b**
TGCAGCGTTGGG**A**C**AGG**GGGAACTAATGAAAAACTGTTTTTT (SEQ ID NO: 127)
**Seq-ID3.5b**
TGCAGCGTTGGG**T**C**CGG**GGG**C**ACTAATGAAAAACTGTTTTTT (SEQ ID NO: 144)
**Seq-ID3.6b**
TGCAGCGT**C**GG**AA**C**AGG**GGG**G**ACTAATGAAAAACTGTTTTTT (SEQ ID NO: 89)
**Seq-ID3.7b**
TGCAGCGTTGGG**A**C**AGG**GGGAACTAATGAAAAACTGTTTTTT (SEQ ID NO: 127)
**Cluster 3 Block J/C** 
GGCAGTGGAACCCAGCTCTCTGTCTTGGAGGACCTGAACAAGGTGTTCCCACCCGAGGTCGCTGTGTTTGAGC
CATCAGAAGCAGAGATCTCCCACACCCAAAAGGCCACACTGGTGTGCCTGGCCACAGGCTTCTTCCCTGACCA
CGTGGAGCTGAGCTGGTGGGTGAATGGGAAGGAGGTGCACAGTGGGGTCAGCACGGACCCGCAGCCCCTCA
AGGAGCAGCCCGCCCTCAATGACTCCAGATACTGCCTGAGCAGCCGCCTGAGGGTCTCGGCCACCTTCTGGCA
GAACCCCCGCAACCACTTCCGCTGTCAAGTCCAGTTCTACGGGCTCTCGGAGAATGACGAGTGGACCCAGGAT
AGGGCCAAACCCGTCACCCAGATCGTCAGCGCCGAGGCCTGGGGTAGAGCAGACTGTGGCTTTACCTCGGTG
TCCTACCAGCAAGGGGTCCTGTCTGCCACCATCCTCTATGAGATCCTGCTAGGGAAGGCCACCCTGTATGCTGT
GCTGGTCAGCGCCCTTGTGTTGATGGCCATGGTCAAGAGAAAGGATTTCTGA (SEQ ID NO: 81)
 **Cluster 3 Block V** 
MLSLLLLLLGLGSVFSAVISQKPSRDICQRGTSLTIQCQVDSQVTMMFWYRQQPGQSLTLIATANQGSEATYESGFV
IDKFPISRPNLTFSTLTVSNMSPEDSSIYL (SEQ ID NO: 16)
 **Seq-ID3.1b**
CSVGAQGTNEKLFF (SEQ ID NO: 15)
**Seq-ID3.2b**
CSVG**SG**GTNEKLFF (SEQ ID NO: 27)
**Seq-ID3.3b**
CSVG**TG**GTNEKLFF (SEQ ID NO: 31)
**Seq-ID3.4b**
CSVG**TG**GTNEKLFF (SEQ ID NO: 31)
**Seq-ID3.5b**
CSVG**SG**GTNEKLFF (SEQ ID NO: 27)
**Seq-ID3.6b**
CSVG**TG**GTNEKLFF (SEQ ID NO: 31)
**Seq-ID3.7b**
CSVG**TG**GTNEKLFF (SEQ ID NO: 31)
**Cluster 3 Block J/C** 
GSGTQLSVLEDLNKVFPPEVAVFEPSEAEISHTQKATLVCLATGFFPDHVELSWWVNGKEVHSGVSTDPQPLKEQP
ALNDSRYCLSSRLRVSATFWQNPRNHFRCQVQFYGLSENDEWTQDRAKPVTQIVSAEAWGRADCGFTSVSYQQG
VLSATILYEILLGKATLYAVLVSALVLMAMVKRKDF (SEQ ID NO: 22)
 Alpha-chain, TRA: **TRAV5^{∗}01, TRAJ23^{∗}01/TRAJ37^{∗}01/TRAJ31^{∗}01, TRAC^{∗}01**
**Cluster 3 Block V** 
ATGAAGACATTTGCTGGATTTTCGTTCCTGTTTTTGTGGCTGCAGCTGGACTGTATGAGTAGAGGAGAGGATG
TGGAGCAGAGTCTTTTCCTGAGTGTCCGAGAGGGAGACAGCTCCGTTATAAACTGCACTTACACAGACAGCTC
CTCCACCTACTTATACTGGTATAAGCAAGAACCTGGAGCAGGTCTCCAGTTGCTGACGTATATTTTTTCAAATAT
GGACATGAAACAAGACCAAAGACTCACTGTTCTATTGAATAAAAAGGATAAACATCTGTCTCTGCGCATTGCA
GACACCCAGACTGGGGACTCAGCTATCTACTTC (SEQ ID NO: 104)
 **Seq-ID3.1a**
TGTGCAGAGAGTACCTCCAGGGGAAAGCTTATCTTC (SEQ ID NO: 100)
**Seq-ID3.2a**
TGTGCAGAGAGTAC**TC**CG**G**G**A**GGAAAGCTTATCTTC (SEQ ID NO: 155)
**Seq-ID3.3a**
TGTGCAGAGAG**CT**C**GC**C**GCAA**GG**C**AA**A**CT**A**ATCTTT (SEQ ID NO: 142)
**Seq-ID3.4a**
TGTGCAGAG**TCA**AC**TC**CC**C**GGGG**C**A**GA**CT**C**AT**G**TT**T** (SEQ ID NO: 110)
**Seq-ID3.5a**
TGTGCAGAGAGTAC**TC**CG**G**G**A**GGAAAGCTTATCTTC (SEQ ID NO: 155)
**Seq-ID3.6a**
TGTGCAGAGAG**CT**C**GC**C**GCAA**GG**C**AA**A**CT**A**ATCTT**T** (SEQ ID NO: 142)
**Seq-ID3.7a**
TGTGCAGAG**TCA**AC**TC**CC**C**GGGG**C**A**GA**CT**C**AT**G**TT**T** (SEQ ID NO: 110)
**Cluster 3 Block J/C** 
GGACAGGGAACGGAGTTATCTGTGAAACCCAATATCCAGAACCCTGACCCTGCCGTGTACCAGCTGAGAGACT
CTAAATCCAGTGACAAGTCTGTCTGCCTATTCACCGATTTTGATTCTCAAACAAATGTGTCACAAAGTAAGGATT
CTGATGTGTATATCACAGACAAAACTGTGCTAGACATGAGGTCTATGGACTTCAAGAGCAACAGTGCTGTGGC
CTGGAGCAACAAATCTGACTTTGCATGTGCAAACGCCTTCAACAACAGCATTATTCCAGAAGACACCTTCTTCC
CCAGCCCAGAAAGTTCCTGTGATGTCAAGCTGGTCGAGAAAAGCTTTGAAACAGATACGAACCTAAACTTTCA
AAACCTGTCAGTGATTGGGTTCCGAATCCTCCTCCTGAAAGTGGCCGGGTTTAATCTGCTCATGACGCTGCGGC
TGTGGTCCAGCTGA (SEQ ID NO: 87)
 **Cluster 3 Block V** 
MKTFAGFSFLFLWLQLDCMSRGEDVEQSLFLSVREGDSSVINCTYTDSSSTYLYWYKQEPGAGLQLLTYIFSNMDM
KQDQRLTVLLNKKDKHLSLRIADTQTGDSAIYF (SEQ ID NO: 57)
 **Seq-ID3.1a**
CAESTSRGKLIF (SEQ ID NO: 24)
**Seq-ID3.2a**
CAEST**PG**GKLIF (SEQ ID NO: 2)
**Seq-ID3.3a**
CAES**SPQ**GKLIF (SEQ ID NO: 21)
**Seq-ID3.4a**
CAEST**P**RG**R**LMF (SEQ ID NO: 32)
**Seq-ID3.5a**
CAEST**PG**GKLIF (SEQ ID NO: 2)
**Seq-ID3.6a**
CAES**SPQ**GKLIF (SEQ ID NO: 21)
**Seq-ID3.7a**
CAEST**P**RG**R**L**M**F (SEQ ID NO: 32)
**Cluster 3 Block J/C**

GQGTELSVKPNIQNPDPAVYQLRDSKSSDKSVCLFTDFDSQTNVSQSKDSDVYITDKTVLDMRSMDFKSNSAVAW
SNKSDFACANAFNNSIIPEDTFFPSPESSCDVKLVEKSFETDTNLNFQNLSVIGFRILLLKVAGFNLLMTLRLWSS
(SEQ ID NO: 4)

**Table 6: Cluster ID 4**

| SEQ-ID | CDR3 **beta:** Amino acid seq. | CDR3 **alpha:** Amino acid seq. | HLA_C_I | HLA_C_II |
|---|---|---|---|---|
| 4.1 | CASSYDSGTGELFF (SEQ ID NO: 9) | CALSETGNQFYF (SEQ ID NO: 52) | | C*07:01 |
| 4.2 | CASSYDSGTGELFF (SEQ ID NO: 9) | CALSETGNQFYF (SEQ ID NO: 52) | | C*07:01 |
| 4.3 | CASSYDSGTGELFF (SEQ ID NO: 9) | CALSDTGNQFYF (SEQ ID NO: 29) | C*07:01 | |
| 4.4 | CASSYDSGTGELFF (SEQ ID NO: 9) | CALSDTGNQFYF (SEQ ID NO: 29) | C*07:01 | |

### Cluster ID 4:

Cluster 4 is associated with **HLA-C^{∗}07:01**
Beta chain, TRB: **TRBV6-5^{∗}01/TRBV6-1^{∗}01, TRBJ2-2^{∗}01, TRBC2^{∗}01**
**Cluster 4 Block V** 
ATGAGCATCGGCCTCCTGTGCTGTGCAGCCTTGTCTCTCCTGTGGGCAGGTCCAGTGAATGCTGGTGTCACTCA
GACCCCAAAATTCCAGGTCCTGAAGACAGGACAGAGCATGACACTGCAGTGTGCCCAGGATATGAACCATGA
ATACATGTCCTGGTATCGACAAGACCCAGGCATGGGGCTGAGGCTGATTCATTACTCAGTTGGTGCTGGTATC
ACTGACCAAGGAGAAGTCCCCAATGGCTACAATGTCTCCAGATCAACCACAGAGGATTTCCCGCTCAGGCTGC
TGTCGGCTGCTCCCTCCCAGACATCTGTGTACTTC (SEQ ID NO: 154)
 **Seq-ID4.1b**
TGTGCCAGCAGTTATGACAGCGGAACCGGGGAGCTGTTTTTT (SEQ ID NO: 121)
**Seq-ID4.2b**
TGTGCCAGCAGTTA**C**GACAG**T**GG**G**ACCGGGGAGCTGTTTTTT (SEQ ID NO: 140)
**Seq-ID4.3b**
TGTGCCAGCAGTTA**C**GAC**TCA**GG**G**ACCGGGGAGCTGTTTTTT (SEQ ID NO: 141)
**Seq-ID4.4b**
TGTGCCAGCAGTTA**C**GAC**TCA**GG**G**ACCGGGGAGCTGTTTTTT (SEQ ID NO: 141)
**Cluster 4 Block J/C** 
GGAGAAGGCTCTAGGCTGACCGTACTGGAGGACCTGAAAAACGTGTTCCCACCCAAGGTCGCTGTGTTTGAG
CCATCAGAAGCAGAGATCTCCCACACCCAAAAGGCCACACTGGTGTGCCTGGCCACAGGCTTCTACCCCGACC
ACGTGGAGCTGAGCTGGTGGGTGAATGGGAAGGAGGTGCACAGTGGGGTCAGCACAGACCCGCAGCCCCTC
AAGGAGCAGCCCGCCCTCAATGACTCCAGATACTGCCTGAGCAGCCGCCTGAGGGTCTCGGCCACCTTCTGGC
AGAACCCCCGCAACCACTTCCGCTGTCAAGTCCAGTTCTACGGGCTCTCGGAGAATGACGAGTGGACCCAGGA
TAGGGCCAAACCTGTCACCCAGATCGTCAGCGCCGAGGCCTGGGGTAGAGCAGACTGTGGCTTCACCTCCGA
GTCTTACCAGCAAGGGGTCCTGTCTGCCACCATCCTCTATGAGATCTTGCTAGGGAAGGCCACCTTGTATGCCG
TGCTGGTCAGTGCCCTCGTGCTGATGGCCATGGTCAAGAGAAAGGATTCCAGAGGCTAG (SEQ ID NO:
128)
 **Cluster 4 Block V** 
MSIGLLCCAALSLLWAGPVNAGVTQTPKFQVLKTGQSMTLQCAQDMNHEYMSWYRQDPGMGLRLIHYSVGAGI
TDQGEVPNGYNVSRSTTEDFPLRLLSAAPSQTSVYF (SEQ ID NO: 39)
 **Seq-ID4.1b**
CASSYDSGTGELFF (SEQ ID NO: 9)
**Seq-ID4.2b**
CASSYDSGTGELFF (SEQ ID NO: 9)
**Seq-ID4.3b**
CASSYDSGTGELFF (SEQ ID NO: 9)
**Seq-ID4.4b**
CASSYDSGTGELFF (SEQ ID NO: 9)
**Cluster 4 Block J/C** 
GEGSRLTVLEDLKNVFPPKVAVFEPSEAEISHTQKATLVCLATGFYPDHVELSWWVNGKEVHSGVSTDPQPLKEQP
ALNDSRYCLSSRLRVSATFWQNPRNHFRCQVQFYGLSENDEWTQDRAKPVTQIVSAEAWGRADCGFTSESYQQG
VLSATILYEILLGKATLYAVLVSALVLMAMVKRKDSRG (SEQ ID NO: 34)
 Alpha-chain, TRA: **TRAV19^{∗}01, TRAJ49^{∗}01, TRAC^{∗}01**
**Cluster 4 Block V** 
ATGCTGACTGCCAGCCTGTTGAGGGCAGTCATAGCCTCCATCTGTGTTGTATCCAGCATGGCTCAGAAGGTAA
CTCAAGCGCAGACTGAAATTTCTGTGGTGGAGAAGGAGGATGTGACCTTGGACTGTGTGTATGAAACCCGTG
ATACTACTTATTACTTATTCTGGTACAAGCAACCACCAAGTGGAGAATTGGTTTTCCTTATTCGTCGGAACTCTT
TTGATGAGCAAAATGAAATAAGTGGTCGGTATTCTTGGAACTTCCAGAAATCCACCAGTTCCTTCAACTTCACC
ATCACAGCCTCACAAGTCGTGGACTCAGCAGTATACTTC (SEQ ID NO: 149)
 **Seq-ID4.1a**
TGTGCTCTGAGTGAAACCGGTAACCAGTTCTATTTT (SEQ ID NO: 114)
**Seq-ID4.2a**
TGTGCTCTGAGTGA**G**ACCGGTAACCAGTTCTATTTT (SEQ ID NO: 91)
**Seq-ID4.3a**
TGTGCTCTGAGTGA**C**ACCGGTAACCAGTTCTATTTT (SEQ ID NO: 99)
**Seq-ID4.4a**
TGTGCTCTGAGTGACACCGGTAACCAGTTCTATTTT (SEQ ID NO: 99)
**Cluster 4 Block J/C** 
GGGACAGGGACAAGTTTGACGGTCATTCCAAATATCCAGAACCCTGACCCTGCCGTGTACCAGCTGAGAGACT
CTAAATCCAGTGACAAGTCTGTCTGCCTATTCACCGATTTTGATTCTCAAACAAATGTGTCACAAAGTAAGGATT
CTGATGTGTATATCACAGACAAAACTGTGCTAGACATGAGGTCTATGGACTTCAAGAGCAACAGTGCTGTGGC
CTGGAGCAACAAATCTGACTTTGCATGTGCAAACGCCTTCAACAACAGCATTATTCCAGAAGACACCTTCTTCC
CCAGCCCAGAAAGTTCCTGTGATGTCAAGCTGGTCGAGAAAAGCTTTGAAACAGATACGAACCTAAACTTTCA
 
AAACCTGTCAGTGATTGGGTTCCGAATCCTCCTCCTGAAAGTGGCCGGGTTTAATCTGCTCATGACGCTGCGGC
TGTGGTCCAGCTGA (SEQ ID NO: 76)
 **Cluster 4 Block V** 
MLTASLLRAVIASICVVSSMAQKVTQAQTEISVVEKEDVTLDCVYETRDTTYYLFWYKQPPSGELVFLIRRNSFDEQN
EISGRYSWNFQKSTSSFNFTITASQVVDSAVYF (SEQ ID NO: 49)
 **Seq-ID4.1a**
CALSETGNQFYF (SEQ ID NO: 52)
**Seq-ID4.2a**
CALSETGNQFYF (SEQ ID NO: 52)
**Seq-ID4.3a**
CALSDTGNQFYF (SEQ ID NO: 29)
**Seq-ID4.4a**
CALSDTGNQFYF (SEQ ID NO: 29)
**Cluster 4 Block J/C**

GTGTSLTVIPNIQNPDPAVYQLRDSKSSDKSVCLFTDFDSQTNVSQSKDSDVYITDKTVLDMRSMDFKSNSAVAWS
NKSDFACANAFNNSIIPEDTFFPSPESSCDVKLVEKSFETDTNLNFQNLSVIGFRILLLKVAGFNLLMTLRLWSS
(SEQ ID NO: 66)

**Table 7: Cluster ID 5**

| SEQ-ID | CDR3 **beta:** Amino acid seq. | CDR3 **alpha:** Amino acid seq. | HLA_A_I | HLA_A_II | HLA_C_I |
|---|---|---|---|---|---|
| 5.1 | CASSLEGQASSYEQYF (SEQ ID NO: 61) | CAGSGAGSYQLTF (SEQ ID NO: 13) | A*01:01 | A*02:01 | C*02:02 |
| 5.2 | CASSLEGQASSYEQYF (SEQ ID NO: 61) | CAGAGAGSYQLTF (SEQ ID NO: 65) | A*01:01 | A*02:01 | C*02:02 |

### Cluster ID 5:

Cluster 5 is associated with the **HLA-A*01:01, HLA-A*02:01 or HLA-C*02:02**
Beta chain, TRB: **TRBV5-1*01, TRBJ2-7*01, TRBC2*01**
**Cluster 5 Block V** 
ATGGGCTCCAGGCTGCTCTGTTGGGTGCTGCTTTGTCTCCTGGGAGCAGGCCCAGTAAAGGCTGGAGTCACTC
AAACTCCAAGATATCTGATCAAAACGAGAGGACAGCAAGTGACACTGAGCTGCTCCCCTATCTCTGGGCATAG
GAGTGTATCCTGGTACCAACAGACCCCAGGACAGGGCCTTCAGTTCCTCTTTGAATACTTCAGTGAGACACAG
AGAAACAAAGGAAACTTCCCTGGTCGATTCTCAGGGCGCCAGTTCTCTAACTCTCGCTCTGAGATGAATGTGA
GCACCTTGGAGCTGGGGGACTCGGCCCTTTATCTT (SEQ ID NO: 116)
 **Seq-ID5.1b**
TGCGCCAGCAGCTTGGAAGGACAGGCGAGCTCCTACGAGCAGTACTTC (SEQ ID NO: 75)
**Seq-ID5.2b**
TGCGCCAGCAGCTTGGA**G**GG**T**CAGGC**C**AGCTCCTACGAGCAGTACTTC (SEQ ID NO: 156)
**Cluster 5 Block J/C** 
GGGCCGGGCACCAGGCTCACGGTCACAGAGGACCTGAAAAACGTGTTCCCACCCAAGGTCGCTGTGTTTGAG
CCATCAGAAGCAGAGATCTCCCACACCCAAAAGGCCACACTGGTGTGCCTGGCCACAGGCTTCTACCCCGACC
ACGTGGAGCTGAGCTGGTGGGTGAATGGGAAGGAGGTGCACAGTGGGGTCAGCACAGACCCGCAGCCCCTC
AAGGAGCAGCCCGCCCTCAATGACTCCAGATACTGCCTGAGCAGCCGCCTGAGGGTCTCGGCCACCTTCTGGC
AGAACCCCCGCAACCACTTCCGCTGTCAAGTCCAGTTCTACGGGCTCTCGGAGAATGACGAGTGGACCCAGGA
TAGGGCCAAACCTGTCACCCAGATCGTCAGCGCCGAGGCCTGGGGTAGAGCAGACTGTGGCTTCACCTCCGA
GTCTTACCAGCAAGGGGTCCTGTCTGCCACCATCCTCTATGAGATCTTGCTAGGGAAGGCCACCTTGTATGCCG
TGCTGGTCAGTGCCCTCGTGCTGATGGCCATGGTCAAGAGAAAGGATTCCAGAGGCTAG (SEQ ID NO: 80)
 **Cluster 5 Block V** 
MGSRLLCWVLLCLLGAGPVKAGVTQTPRYLIKTRGQQVTLSCSPISGHRSVSWYQQTPGQGLQFLFEYFSETQRNK
GNFPGRFSGRQFSNSRSEMNVSTLELGDSALYL (SEQ ID NO: 43)
 **Seq-ID5.1b**
CASSLEGQASSYEQYF (SEQ ID NO: 61)
**Seq-ID5.2b**
CASSLEGQASSYEQYF (SEQ ID NO: 61)
**Cluster 5 Block J/C** 
GPGTRLTVTEDLKNVFPPKVAVFEPSEAEISHTQKATLVCLATGFYPDHVELSWWVNGKEVHSGVSTDPQPLKEQP
ALNDSRYCLSSRLRVSATFWQNPRNHFRCQVQFYGLSENDEWTQDRAKPVTQIVSAEAWGRADCGFTSESYQQG
VLSATILYEILLGKATLYAVLVSALVLMAMVKRKDSRG (SEQ ID NO: 69)
 Alpha-chain, TRA: **TRAV25^{∗}01, TRAJ28^{∗}01, TRAC^{∗}01**
**Cluster 5 Block V** 
ATGCTACTCATCACATCAATGTTGGTCTTATGGATGCAATTGTCACAGGTGAATGGACAACAGGTAATGCAAAT
TCCTCAGTACCAGCATGTACAAGAAGGAGAAGACTTCACCACGTACTGCAATTCCTCAACTACTTTAAGCAATA
TACAGTGGTATAAGCAAAGGCCTGGTGGACATCCCGTTTTTTTGATACAGTTAGTGAAGAGTGGAGAAGTGAA
GAAGCAGAAAAGACTGACATTTCAGTTTGGAGAAGCAAAAAAGAACAGCTCCCTGCACATCACAGCCACCCA
GACTACAGATGTAGGAACCTACTTC (SEQ ID NO: 105)
 **Seq-ID5.1a**
TGTGCAGGATCTGGGGCTGGGAGTTACCAACTCACTTTC (SEQ ID NO: 126)
**Seq-ID5.2a**
GGC**T**GG**GG**CTGGGAGTTACCAACTCACTTTC (SEQ ID NO: 135)
**Cluster 5 Block J/C** 
GGGAAGGGGACCAAACTCTCGGTCATACCAAATATCCAGAACCCTGACCCTGCCGTGTACCAGCTGAGAGACT
CTAAATCCAGTGACAAGTCTGTCTGCCTATTCACCGATTTTGATTCTCAAACAAATGTGTCACAAAGTAAGGATT
CTGATGTGTATATCACAGACAAAACTGTGCTAGACATGAGGTCTATGGACTTCAAGAGCAACAGTGCTGTGGC
CTGGAGCAACAAATCTGACTTTGCATGTGCAAACGCCTTCAACAACAGCATTATTCCAGAAGACACCTTCTTCC
CCAGCCCAGAAAGTTCCTGTGATGTCAAGCTGGTCGAGAAAAGCTTTGAAACAGATACGAACCTAAACTTTCA
AAACCTGTCAGTGATTGGGTTCCGAATCCTCCTCCTGAAAGTGGCCGGGTTTAATCTGCTCATGACGCTGCGGC
TGTGGTCCAGCTGA (SEQ ID NO: 153)
 **Cluster 5 Block V** 
MLLITSMLVLWMQLSQVNGQQVMQIPQYQHVQEGEDFTTYCNSSTTLSNIQWYKQRPGGHPVFLIQLVKSGEVK
KQKRLTFQFGEAKKNSSLHITATQTTDVGTYF (SEQ ID NO: 35)
 **Seq-ID5.1a**
CAGSGAGSYQLTF (SEQ ID NO: 13)
**Seq-ID5.2a**
CAG**A**GAGSYQLTF (SEQ ID NO: 65)
**Cluster 5 Block J/C**

GKGTKLSVIPNIQNPDPAVYQLRDSKSSDKSVCLFTDFDSQTNVSQSKDSDVYITDKTVLDMRSMDFKSNSAVAWS
NKSDFACANAFNNSIIPEDTFFPSPESSCDVKLVEKSFETDTNLNFQNLSVIGFRILLLKVAGFNLLMTLRLWSS
(SEQ ID NO: 40)

**Table 8: Cluster ID 6**

| SEQ-ID | CDR3 **beta:** Amino acid seq. | CDR3 **alpha:** Amino acid seq. | HLA_A_I |
|---|---|---|---|
| 6.1 | CASSIGSGSYNEQFF (SEQ ID NO: 10) | CVVSAGREYGNKLVF (SEQ ID NO: 25) | B*15:01 |
| 6.2 | CASSLTSGNYNEQFF (SEQ ID NO: 42) | CVVSAGREYGNKLVF (SEQ ID NO: 25) | B*15:01 |
| 6.3 | CASSRTSGSLNEQFF (SEQ ID NO: 7) | CVVTAGREYGNKLVF (SEQ ID NO: 5) | B*15:01 |
| 6.4 | CASTVTSGSYNEQFF (SEQ ID NO: 44) | CVVSAGREYGNKLVF (SEQ ID NO: 25) | B*15:01 |
| 6.5 | CASSLTSGSYNEQFF (SEQ ID NO: 17) | CVVSVGREYGNKLVF (SEQ ID NO: 46) | B*15:01 |

### Cluster ID 6:

Cluster 6 is associated with the **HLA-B*15:01**
**Beta chain, TRB: TRBV19^{∗}01, TRBJ2-1^{∗}01, TRBC2^{∗}01**
**Cluster 6 Block V** 
ATGAGCAACCAGGTGCTCTGCTGTGTGGTCCTTTGTTTCCTGGGAGCAAACACCGTGGATGGTGGAATCACTC
AGTCCCCAAAGTACCTGTTCAGAAAGGAAGGACAGAATGTGACCCTGAGTTGTGAACAGAATTTGAACCACGA
TGCCATGTACTGGTACCGACAGGACCCAGGGCAAGGGCTGAGATTGATCTACTACTCACAGATAGTAAATGAC
TTTCAGAAAGGAGATATAGCTGAAGGGTACAGCGTCTCTCGGGAGAAGAAGGAATCCTTTCCTCTCACTGTGA
CATCGGCCCAAAAGAACCCGACAGCTTTCTATCTC (SEQ ID NO: 109)
**Seq-ID6.1b**
   TGTGCCAGTAGTATTGGCAGCGGGAGTTACAATGAGCAGTTCTTC (SEQ ID NO: 118)
**Seq-ID6.2b**
   TGTG**TGGTG**AG**CGCC**GG**G**AG**G**G**AATA**T**GGA**AA**CA**A**A**C**T**G**G**TCTT**T** (SEQ ID NO: 94)
**Seq-ID6.3b**
   TGTGCCAGTAGT**CGGACT**AGCGGGAGT**CTT**AATGAGCAGTTCTTC (SEQ ID NO: 93)
**Seq-ID6.4b**
   TGTGCCAGTA**CCGTAACAA**GCGGGAG**C**TACAATGAGCAGTTCTTC(SEQ ID NO: 117)
**Seq-ID6.5b**
   TGTGCCAGTAGT**C**T**CACT**AGCGG**TTCC**TACAATGAGCAGTTCTTC (SEQ ID NO: 95)
**Cluster 6 Block J/C** 
GGGCCAGGGACACGGCTCACCGTGCTAGAGGACCTGAAAAACGTGTTCCCACCCAAGGTCGCTGTGTTTGAG
CCATCAGAAGCAGAGATCTCCCACACCCAAAAGGCCACACTGGTGTGCCTGGCCACAGGCTTCTACCCCGACC
ACGTGGAGCTGAGCTGGTGGGTGAATGGGAAGGAGGTGCACAGTGGGGTCAGCACAGACCCGCAGCCCCTC
AAGGAGCAGCCCGCCCTCAATGACTCCAGATACTGCCTGAGCAGCCGCCTGAGGGTCTCGGCCACCTTCTGGC
AGAACCCCCGCAACCACTTCCGCTGTCAAGTCCAGTTCTACGGGCTCTCGGAGAATGACGAGTGGACCCAGGA
TAGGGCCAAACCTGTCACCCAGATCGTCAGCGCCGAGGCCTGGGGTAGAGCAGACTGTGGCTTCACCTCCGA
GTCTTACCAGCAAGGGGTCCTGTCTGCCACCATCCTCTATGAGATCTTGCTAGGGAAGGCCACCTTGTATGCCG
TGCTGGTCAGTGCCCTCGTGCTGATGGCCATGGTCAAGAGAAAGGATTCCAGAGGCTAG (SEQ ID NO:
113)
**Cluster 6 Block V** 
MSNQVLCCVVLCFLGANTVDGGITQSPKYLFRKEGQNVTLSCEQNLNHDAMYWYRQDPGQGLRLIYYSQIVNDF
QKGDIAEGYSVSREKKESFPLTVTSAQKNPTAFYL (SEQ ID NO: 36)
**Seq-ID6.1b**
   CASSIGSGSYNEQFF (SEQ ID NO: 10)
**Seq-ID6.2b**
   CASS**LT**SG**N**YNEQFF (SEQ ID NO: 42)
**Seq-ID6.3b**
   CASS**RT**SGS**L**NEQFF (SEQ ID NO: 7)
**Seq-ID6.4b**
   CAS**TVT**SGSYNEQFF (SEQ ID NO: 44)
**Seq-ID6.5b**
   CASS**LT**SGSYNEQFF (SEQ ID NO: 17)
**Cluster 6 Block J/C** 
GPGTRLTVLEDLKNVFPPKVAVFEPSEAEISHTQKATLVCLATGFYPDHVELSWWVNGKEVHSGVSTDPQPLKEQP
ALNDSRYCLSSRLRVSATFWQNPRNHFRCQVQFYGLSENDEWTQDRAKPVTQIVSAEAWGRADCGFTSESYQQG
VLSATILYEILLGKATLYAVLVSALVLMAMVKRKDSRG (SEQ ID NO: 56)
**Alpha-chain, TRA: TRAV10*01, TRAJ47^{∗}**0**1, TRAC^{∗}01**
**Cluster 6 Block V** 
ATGAAAAAGCATCTGACGACCTTCTTGGTGATTTTGTGGCTTTATTTTTATAGGGGGAATGGCAAAAACCAAGT
GGAGCAGAGTCCTCAGTCCCTGATCATCCTGGAGGGAAAGAACTGCACTCTTCAATGCAATTATACAGTGAGC
CCCTTCAGCAACTTAAGGTGGTATAAGCAAGATACTGGGAGAGGTCCTGTTTCCCTGACAATCATGACTTTCAG
TGAGAACACAAAGTCGAACGGAAGATATACAGCAACTCTGGATGCAGACACAAAGCAAAGCTCTCTGCACATC
ACAGCCTCCCAGCTCAGCGATTCAGCCTCCTACATC (SEQ ID NO: 150)
**Seq-ID6.1a**
   TGTGTGGTGAGCGCGGGGAGGGAATATGGAAACAAACTGGTCTTT (SEQ ID NO: 122)
**Seq-ID6.2a**
   TGTGTGGTGAGCGC**C**GGGAGGGAATATGGAAACAAACTGGTCTTT (SEQ ID NO: 94)
**Seq-ID6.3a**
   TGTGTGGTGA**C**CGCGGGGAGGGAATATGGAAACAAACTGGTCTTT (SEQ ID NO: 130)
**Seq-ID6.4a**
   TGTGTGGTGAGCGCGGGGAGGGAATATGGAAACAAACTGGTCTTT (SEQ ID NO: 122)
**Seq-ID6.5a**
   TGTGTGGTGAGCG**TT**GG**A**AGGGAATATGGAAACAAACTGGTCTTT (SEQ ID NO: 82)
**Cluster 6 Block J/C** 
GGCGCAGGAACCATTCTGAGAGTCAAGTCCTATATCCAGAACCCTGACCCTGCCGTGTACCAGCTGAGAGACT
CTAAATCCAGTGACAAGTCTGTCTGCCTATTCACCGATTTTGATTCTCAAACAAATGTGTCACAAAGTAAGGATT
CTGATGTGTATATCACAGACAAAACTGTGCTAGACATGAGGTCTATGGACTTCAAGAGCAACAGTGCTGTGGC
CTGGAGCAACAAATCTGACTTTGCATGTGCAAACGCCTTCAACAACAGCATTATTCCAGAAGACACCTTCTTCC
CCAGCCCAGAAAGTTCCTGTGATGTCAAGCTGGTCGAGAAAAGCTTTGAAACAGATACGAACCTAAACTTTCA
AAACCTGTCAGTGATTGGGTTCCGAATCCTCCTCCTGAAAGTGGCCGGGTTTAATCTGCTCATGACGCTGCGGC
TGTGGTCCAGCTGA (SEQ ID NO: 137)
**Cluster 6 Block V** 
MKKHLTTFLVILWLYFYRGNGKNQVEQSPQSLIILEGKNCTLQCNYTVSPFSNLRWYKQDTGRGPVSLTIMTFSENT
KSNGRYTATLDADTKQSSLHITASQLSDSASYI (SEQ ID NO: 14)
**Seq-ID6.1a**
   CVVSAGREYGNKLVF (SEQ ID NO: 25)
**Seq-ID6.2a**
   CVVSAGREYGNKLVF (SEQ ID NO: 25)
**Seq-ID6.3a**
   CVV**T**AGREYGNKLVF (SEQ ID NO: 5)
**Seq-ID6.4a**
   CVVSAGREYGNKLVF (SEQ ID NO: 25)
**Seq-ID6.5a**
   CVVS**V**GREYGNKLVF (SEQ ID NO: 46)
**Cluster 6 Block J/C**

GAGTILRVKSYIQNPDPAVYQLRDSKSSDKSVCLFTDFDSQTNVSQSKDSDVYITDKTVLDMRSMDFKSNSAVAWS
NKSDFACANAFNNSIIPEDTFFPSPESSCDVKLVEKSFETDTNLNFQNLSVIGFRILLLKVAGFNLLMTLRLWSS (SEQ
ID NO: 50)

## Claims

1. An isolated T cell receptor (TCR), wherein the TCR comprises a CDR3 alpha sequence and a CDR3 beta sequence, wherein the CDR3 alpha sequence and the CDR3 beta sequence are identical to the sequences given below, or with one or two amino acid substitutions per CDR3 sequence,
wherein
a. for group a, the CDR3 alpha sequence is selected from the group of sequences comprising SEQ ID NO 6, SEQ ID NO 1, SEQ ID NO 59, and the CDR3 beta sequence is selected from the group of sequences comprising SEQ ID NO 38, SEQ ID NO 41, SEQ ID NO 20, or
b. for group b, the CDR3 alpha sequence is selected from the group of sequences comprising SEQ ID NO 11, SEQ ID NO 12, SEQ ID NO 18, SEQ ID NO 26, SEQ ID NO 55, SEQ ID NO 58, and the CDR3 beta sequence is selected from the group of sequences comprising SEQ ID NO 47, SEQ ID NO 72, SEQ ID NO 73, or
c. for group c, the CDR3 alpha sequence is selected from the group of sequences comprising SEQ ID NO 62, SEQ ID NO 63, SEQ ID NO 71, and the CDR3 beta sequence is selected from the sequence SEQ ID NO 28, or
d. for group d, the CDR3 alpha sequence is selected from the group of sequences comprising SEQ ID NO 8, SEQ ID NO 51, and the CDR3 beta sequence is selected from the group of sequences comprising SEQ ID NO 19, SEQ ID NO 45, SEQ ID NO 60, or
e. for group e, the CDR3 alpha sequence is selected from the group of sequences comprising SEQ ID NO 2, SEQ ID NO 21, SEQ ID NO 24, SEQ ID NO 32, and the CDR3 beta sequence is selected from the group of sequences comprising SEQ ID NO 15, SEQ ID NO 27, SEQ ID NO 31, or
f. for group f, the CDR3 alpha sequence is selected from the group of sequences comprising SEQ ID NO 29, SEQ ID NO 52, and the CDR3 beta sequence is selected from the sequence SEQ ID NO 9, or
g. for group g, the CDR3 alpha sequence is selected from the group of sequences comprising SEQ ID NO 13, SEQ ID NO 65, and the CDR3 beta sequence is selected from the sequence SEQ ID NO 61, or
h. for group h, the CDR3 alpha sequence is selected from the group of sequences comprising SEQ ID NO 5, SEQ ID NO 25, SEQ ID NO 46, and the CDR3 beta sequence is selected from the group of sequences comprising SEQ ID NO 7, SEQ ID NO 10, SEQ ID NO 17, SEQ ID NO 42, SEQ ID NO 44,
particularly wherein the CRD3 alpha sequence and the CDR3 beta sequence are identified in the same row of tables 1-8,
particularly wherein said substitutions are selected according to the substitution rules given below,
wherein the substitution rules are:
- glycine (G) and alanine (A) are interchangeable; valine (V), leucine (L), and isoleucine (I) are interchangeable, A and V are interchangeable;
- tryptophan (W) and phenylalanine (F) are interchangeable, tyrosine (Y) and F are interchangeable;
- serine (S) and threonine (T) are interchangeable;
- aspartic acid (D) and glutamic acid (E) are interchangeable
- asparagine (N) and glutamine (Q) are interchangeable; N and S are interchangeable; N and D are interchangeable; E and Q are interchangeable;
- methionine (M) and Q are interchangeable;
- cysteine (C), A and S are interchangeable;
- proline (P), G and A are interchangeable;
- arginine (R) and lysine (K) are interchangeable.

2. The isolated TCR according to claim 1, wherein the CDR3 sequences are selected from the groups a, b, f, g, and h.

3. The isolated TCR according to claim 1 or 2, wherein the TCR additionally comprises a variable (V) alpha sequence, a joining-constant (JC) alpha sequence, a V beta sequence, and a JC beta sequence or a sequence with ≥80%, ≥85%, ≥90%, ≥92%, ≥94%, ≥96%, ≥98%, or ≥99% sequence identity to said sequences,
wherein
a. for group a, the V alpha sequence is SEQ ID NO: 67, the JC alpha sequence is SEQ ID NO: 4, the V beta sequence is SEQ ID NO: 23, and the JC beta sequence is SEQ ID NO: 33, or
b. for group b, the V alpha sequence is SEQ ID NO: 3, the JC alpha sequence is SEQ ID NO: 53, the V beta sequence is SEQ ID NO: 64, and the JC beta sequence is SEQ ID NO: 56, or
c. for group c, the V alpha sequence is SEQ ID NO: 54, the JC alpha sequence is SEQ ID NO: 48, the V beta sequence is SEQ ID NO: 43, and the JC beta sequence is SEQ ID NO: 37, or
d. for group d, the V alpha sequence is SEQ ID NO: 68, the JC alpha sequence is SEQ ID NO: 70, the V beta sequence is SEQ ID NO: 30, and the JC beta sequence is SEQ ID NO: 37, or
e. for group e, the V alpha sequence is SEQ ID NO: 57, the JC alpha sequence is SEQ ID NO: 4, the V beta sequence is SEQ ID NO: 16, and the JC beta sequence is SEQ ID NO: 22, or
f. for group f, the V alpha sequence is SEQ ID NO: 49, the JC alpha sequence is SEQ ID NO: 66, the V beta sequence is SEQ ID NO: 39, and the JC beta sequence is SEQ ID NO: 34, or
g. for group g, the V alpha sequence is SEQ ID NO: 35, the JC alpha sequence is SEQ ID NO: 40, the V beta sequence is SEQ ID NO: 43, and the JC beta sequence is SEQ ID NO: 69, or
h. for group h, the V alpha sequence is SEQ ID NO: 14, the JC alpha sequence is SEQ ID NO: 50, the V beta sequence is SEQ ID NO: 36, and the JC beta sequence is SEQ ID NO: 56.

4. A nucleic acid sequence encoding the TCR according to any one of claims 1 to 3.

5. An isolated autologous T cell comprising a TCR according to any one of claims 1 to 3, and/or a nucleic acid sequence according to claim 4.

6. The isolated autologous T cell according to claim 5, wherein the isolated autologous T cell is a recombinant T cell.

7. The TCR according to any one of claims 1 to 3, the nucleic acid sequence according to claim 4, or the isolated autologous T cell according to claims 5 to 6 for use in treatment of cancer.

8. The TCR, the nucleic acid sequence, or the isolated autologous T cell for use according to claim 7, wherein the TCR or the nucleic acid or the T cell is administered to a patient with the following HLA-type:
a. HLA-B*08:01 and/or HLA-C*07:01 for group a; or
b. HLA-A*02:01-supertype (HLA-A*02:01/68:02) for group b; or
c. HLA-A*02:01-supertype (HLA-A*02:01/02:35/02:05) and/or HLA-C*07:01/07:04 for group c; or
d. HLA-A*01:01 and/or HLA-A*02:01 for group d; or
e. HLA-A*02:01 and/or the HLA-A*01-supertype (A*01:01/68:01) for group e; or
f. HLA-C*07:01 for group f; or
g. HLA-A*01:01 and/or HLA-A*02:01 and/or HLA-C*02:02 for group g; or
h. HLA-B*15:01 for group h.
